# EUROPÄISCHE PATENTSCHRIFT

(11) **EP 0 640 587 B1**
(45) Veröffentlichungstag und Bekanntmachung des Hinweises auf die Patenterteilung: **29.12.1999**
(21) Anmeldenummer: 94112895.1
(22) Anmeldetag: 18.08.1994
(51) Int. Cl.: C07C 279/20, C07C 279/22, A61K 31/155, C07D 231/12, C07D 295/155, C07D 207/327

(54) **Diacylsubstituierte Guanidine, Verfahren zu ihrer Herstellung, ihre Verwendung als Medikament oder Diagnostikum sowie sie enthaltendes Medikament**
Diacyl substituted guanidins, process for their preparation, their use as medicament or diagnostic reagent and medicament containing them
Guanidines diacyl substitués, procédé de leur préparation, leur utilisation comme médicament ou comme agent diagnostique ainsi que médicaments les contenant

(30) Priorität: 24.08.1993 DE 4328352
(43) Veröffentlichungstag der Anmeldung: 01.03.1995
(60) Teilanmeldung: 99102583.4
(73) Patentinhaber: HOECHST AKTIENGESELLSCHAFT, 65929 Frankfurt am Main (DE)
(72) Erfinder: Kleemann, Heinz-Werner Dr., D-61350 Bad-Homburg (DE); Lang, Hans-Jochen Dr., D-65719 Hofheim (DE); Weichert, Andreas Dr., D-63329 Egelsbach (DE); Crause, Peter Dr., D-63069 Offenbach (DE); Scholz, Wolfgang Dr., D-65760 Eschborn (DE); Albus, Udo Dr., D-61197 Florstadt (DE); Schwark, Jan-Robert Dr., D-65929 Frankfurt (DE)

(56) Entgegenhaltungen:
- WO-A-84/00875
- US-A- 4 225 613
- CHEMICAL ABSTRACTS, vol. 107, no. 13, 1987, Columbus, Ohio, US; abstract no. 111059y, & J. NAT. SCI. MATH., Bd.27, Nr.1, 1987 Seiten 149 - 156 Z.A. MALIK ET AL
- CHEMICAL ABSTRACTS, vol. 68, no. 7, 1968, Columbus, Ohio, US; abstract no. 30007d, & ROCZ. CHEM., Bd.41, Nr.6, 1967 Seiten 1087 - 1091 R. KRUG ET AL

## Beschreibung

Die Erfindung betrifft Di-benzoylguanidine der Formel I a 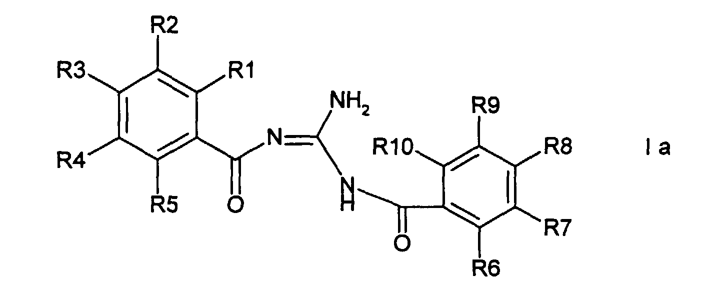 worin bedeuten:
R(1), R(5), R(6) und R(10)
unabhängig voneinander Wasserstoff, F, Cl, (C₁-C₃)-Alkyl, -OR(11), CᵣF_{2r + 1} oder -NR(11)R(12);
R(11) und R(12)
   unabhängig voneinander Wasserstoff oder (C₁-C₃)-Alkyl;
r 1 bis 4;

R(2), R(4), R(7) und R(9)
unabhängig voneinander Wasserstoff, F, Cl, Br, I, -C≡N, Xₒ-(CH₂)ₚ-(CF₂)_{q}-CF₃, R(13)-SOₘ, R(14)R(15)N-CO-, R(16)-CO- oder R(17)R(18)N-SO₂-;
X Sauerstoff, S oder NR(19);
   R(19) Wasserstoff oder (C₁-C₃)-Alkyl;
m Null, 1 oder 2;
o Null oder 1;
p Null, 1 oder 2;
q Null, 1, 2, 3, 4, 5 oder 6;
R(13), R(14), R(16) und R(17)
   unabhängig voneinander (C₁-C₈)-Alkyl, (C₃-C₆)-Alkenyl, -CₙH₂ₙ-R(20) oder CF₃;
   n Null, 1, 2, 3 oder 4;
   R(20) (C₃-C₇)-Cycloalkyl oder Phenyl,
      welches nicht substituiert ist oder substituiert mit 1 - 3 Substituenten aus der Gruppe F, Cl, CF₃, Methyl, Methoxy und NR(21)R(22);
      R(21) und R(22)
         H oder C₁-C₄-Alkyl,
   oder
R(14), R(16) und R(17)
   auch H;
R(15) und R(18)
   unabhängig voneinander Wasserstoff oder (C₁-C₄)-Alkyl;
   oder
R(14) und R(15) sowie R(17) und R(18)
   gemeinsam 4 oder 5 Methylengruppen,
      von denen eine CH₂-Gruppe durch Sauerstoff, S, NH, N-CH₃ oder N-Benzyl ersetzt sein kann;
oder
R(2), R(4), R(7) und R(9)
unabhängig voneinander (C₁-C₈)-Alkyl oder -CₐₗH₂ₐₗR(84);
al Null, 1 oder 2;
R(84) (C₃-C₈)-Cycloalkyl oder Phenyl,
   welches nicht substituiert oder substituiert ist mit 1 - 3 Substituenten aus der Gruppe F, Cl, CF₃, Methyl, Methoxy und NR(85)R(86);
   R(85) und R(86)
      Wasserstoff oder CH₃;
oder
R(2), R(4), R(7) und R(9)
unabhängig voneinander Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl,
   die über C oder N verknüpft sind und die unsubstituiert oder substituiert sind mit 1 - 3 Substituenten aus der Gruppe F, Cl, CF₃, CH₃, Methoxy, Hydroxy, Amino, Methylamino und Dimethylamino;
oder
R(2), R(4), R(7) und R(9)
unabhängig voneinander -C≡CR(93),
R(93) Phenyl,
   das unsubstituiert oder substituiert ist mit 1 - 3 Substituenten aus der Gruppe F, Cl, CF₃, Methyl, Methoxy und NR(94)R(95);
   R(94) und R(95)
      Wasserstoff oder CH₃;

R(3) und R(8)
unabhängig voneinander wie R(2) definiert,
oder
R(3) und R(8)
unabhängig voneinander -Y-(p-C₆H₄)-(CO)ₕ-(CHOH)ᵢ-(CH₂)ⱼ-(-CHOH)ₖ-R(23), -Y-(m-C₆H₄)-(CO)_{ad}-(CHOH)ₐₑ-(CH₂)_{af}-(CHOH)_{ag}-R(24) oder -Y-(o-C₆H₄)-(CO)ₐₕ-(CHOH)ₐₒ-(CH₂)ₐₚ-(CHOH)ₐₖ-R(25),
Y Sauerstoff, -S- oder -NR(83)-;
h, ad, ah
   unabhängig voneinander Null oder 1;
i, j, k, ae, af, ag, ao, ap und ak
   unabhängig voneinander Null, 1, 2, 3 oder 4;
   wobei jedoch jeweils h, i und k nicht gleichzeitig Null,
   ad, ae und ag nicht gleichzeitig Null sowie
   ah, ao und ak nicht gleichzeitig Null sind,
R(23), R(24) R(25) und R(83)
   unabhängig voneinander Wasserstoff oder (C₁-C₃)-Alkyl;
oder
R(3) und R(8)
unabhängig voneinander Wasserstoff, F, Cl, Br, I, CN, (C₁-C₈)-Alkyl, (C₁-C₈)-Perfluoralkyl, (C₃-C₈)-Alkenyl, -C_{g}H_{2g}R(26),
g Null, 1, 2, 3 oder 4;
R(26) (C₃-C₈)-Cycloalkyl, Phenyl, Biphenylyl oder Naphthyl,
   wobei die Aromaten nicht substituiert oder substituiert sind mit 1 - 3 Substituenten aus der Gruppe F, Cl, CF₃, Methyl, Methoxy und NR(27)R(28);
   R(27) und R(28)
      Wasserstoff, (C₁-C₄)-Alkyl oder (C₁-C₄)-Perfluoralkyl;
oder
R(3) und R(8)
unabhängig voneinander SR(29), -OR(30), -NR(31)R(32) oder -CR(33)R(34)R(35);
R(29), R(30), R(31) und R(33)
   unabhängig voneinander -CₐH₂ₐ-[Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl],
      die unsubstituiert oder substituiert sind mit 1 - 3 Substituenten aus der Gruppe F, Cl, CF₃, CH₃, Methoxy, Hydroxy, Amino, Methylamino und Dimethylamino,
   a Null, 1 oder 2;
R(32), R(34) und R(35)
   unabhängig voneinander wie R(29) definiert oder Wasserstoff, (C₁-C₄)-Alkyl oder (C₁-C₄)-Perfluoralkyl;
oder
R(3) und R(8)
unabhängig voneinander -W-(p-C₆H₄)-R(96), -W-(m-C₆H₄)-R(97) oder -W-(o-C₆H₄)-R(98);
R(96), R(97) und R(98)
   unabhängig voneinander Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl,
      die über C oder N verknüpft sind und die unsubstituiert oder substituiert sind mit 1 bis 3 Substituenten aus der Gruppe F, Cl, CF₃, CH₃, Methoxy, Hydroxy, Amino, Methylamino, Dimethylamino oder Benzyl;
W Sauerstoff, S oder NR(99)-;
   R(99) Wasserstoff oder (C₁-C₄)-Alkyl;
oder
R(3) und R(8)
unabhängig voneinander R(72)-SOₘ oder R(73)R(74)N-SO₂-;
m 1 oder 2;
R(72) (C₁-C₈)-Alkyl, (C₁-C₈)-Perfluoralkyl, (C₃-C₈)-Alkenyl oder -CₛH₂ₛ-R(75),
   s Null, 1, 2, 3 oder 4;
   R(75) (C₃-C₈)-Cycloalkyl, Phenyl, Biphenylyl oder Naphthyl,
      wobei die Aromaten nicht substituiert oder substituiert sind mit 1 - 3 Substituenten aus der Gruppe F, Cl, CF₃, Methyl, Methoxy und NR(76)R(77);
      R(76) und R(77)
         Wasserstoff, (C₁-C₄)-Alkyl oder (C₁-C₄)-Perfluoralkyl;
R(73) Wasserstoff, (C₁-C₈)-Alkyl, (C₁-C₈)-Perfluoralkyl, (C₃-C₈)-Alkenyl oder -C_{w}H_{2w}-R(78),
   w Null, 1, 2, 3 oder 4;
   R(78) (C₃-C₈)-Cycloalkyl, Phenyl, Biphenylyl oder Naphthyl,
      wobei die Aromaten nicht substituiert oder substituiert sind mit 1 - 3 Substituenten aus der Gruppe F, Cl, CF₃, Methyl, Methoxy und NR(79)R(80);
      R(79) und R(80)
         Wasserstoff, (C₁-C₄)-Alkyl oder (C₁-C₄)-Perfluoralkyl;
R(74) Wasserstoff, (C₁-C₄)-Alkyl oder (C₁-C₄)-Perfluoralkyl,
   oder
R(73) und R(74)
   gemeinsam 4 oder 5 Methylengruppen, von denen eine CH₂-Gruppe durch Sauerstoff, S, NH, N-CH₃ oder N-Benzyl ersetzt sein kann;
oder
R(3) und R(8)
unabhängig voneinander R(39)X-,
X Sauerstoff, S, NR(40), (D = O)A-, NR(41)C = MN⁽*⁾R(42)-;
   M Sauerstoff oder S;
   A Sauerstoff oder NR(43);
   D C oder SO;
R(39) (C₁-C₈)-Alkyl, (C₃-C₈)-Alkenyl, (CH₂)_{b}C_{d}F_{2d + 1} oder -CₓH₂ₓ-R(44);
   b Null oder 1;
   d 1, 2, 3, 4, 5, 6 oder 7;
   x Null, 1, 2 , 3, oder 4;
   R(44) (C₃-C₈)-Cycloalkyl, Phenyl, Biphenylyl oder Naphthyl,
      wobei die Aromaten nicht substituiert oder substituiert sind mit 1 - 3 Substituenten aus der Gruppe F, Cl, CF₃, Methyl, Methoxy und NR(45)R(46);
      R(45) und R(46)
         Wasserstoff, (C₁-C₄)-Alkyl oder (C₁-C₄)-Perfluoralkyl;
   R(40), R(41) und R(43)
      unabhängig voneinander Wasserstoff, (C₁-C₄)-Alkyl oder (C₁-C₄)-Perfluoralkyl;
   R(42) definiert wie R(39);
      oder
   R(39) und R(40) beziehungsweise R(39) und R(41)
      gemeinsam 4 oder 5 Methylengruppen,
         von denen eine CH₂-Gruppe durch Sauerstoff, S, NH, N-CH₃ oder N-Benzyl ersetzt sein kann;
      wobei A und N⁽*⁾ an den Phenylkern des Benzoylguanidin-Grundkörpers gebunden sind;
oder
R(3) und R(8)
unabhängig voneinander -SR(47), -OR(48), -NHR(49), -NR(50)R(51), -CHR(52)R(53), -CR(54)R(55)OH, -C≡C-R(56), -CR(58) = CHR(57) oder -[CR(59)R(60)]ᵤ-CO-[CR(61)R(62)]ᵥ-R(63);
R(47), R(48), R(49), R(50) und R(52)
   gleich oder verschieden -(CH₂)_{y}-(CHOH)_{z}-(CH₂)ₐₐ-(CHOH)ₜ-R(64) oder -(CH₂)_{ab}-O-(CH₂-CH₂O)_{ac}-R(65);
   R(64) und R(65)
      Wasserstoff oder Methyl;
u 1, 2, 3 oder 4;
v Null, 1, 2, 3 oder 4;
   y, z und aa
      gleich oder verschieden Null, 1, 2, 3 oder 4;
   t = 1, 2, 3 oder 4;
R(51), R(53), R(54) und R(55)
   gleich oder verschieden Wasserstoff oder (C₁-C₆)-Alkyl;
   oder
R(52) und R(53) beziehungsweise R(54) und R(55)
   zusammen mit dem sie tragenden Kohlenstoff-Atom ein (C₃-C₈)-Cycloalkyl;
R(63) Wasserstoff, (C₁-C₆)-Alkyl, (C₃-C₈)-Cycloalkyl oder -CₑH₂ₑ-R(81);
   e Null, 1, 2, 3 oder 4;
R(56), R(57) und R(81)
   unabhängig Phenyl,
      das unsubstituiert oder substituiert ist mit 1 - 3 Substituenten aus der Gruppe F, Cl, CF₃, Methyl, Methoxy oder NR(82)R(66) mit R(82) und R(66) gleich H oder (C₁-C₄)-Alkyl;
   oder
R(56), R(57) und R(81)
   unabhängig voneinander Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl],
      die unsubstituiert oder wie Phenyl substituiert sind;
R(58), R(59), R(60), R(61) und R(62)
   Wasserstoff oder Methyl;
oder
R(3) und R(8)
unabhängig voneinander R(67)-NH-SO₂-;
R(67) R(68)R(69)N-(C = Y')-;
Y' Sauerstoff, S oder N-R(70);
   R(68) und R(69)
      gleich oder verschieden Wasserstoff, (C₁-C₈)-Alkyl, (C₃-C₆)-Alkenyl oder -C_{f}H_{2f}-R(71);
      f Null, 1, 2, 3 oder 4;
      R(71) (C₅-C₇)-Cycloalkyl oder Phenyl,
         welches unsubstituiert ist oder substituiert mit 1 - 3 Substituenten aus der Gruppe F, Cl, CF₃, Methoxy und (C₁-C₄)-Alkyl;
      oder
   R(68) und R(69)
      gemeinsam 4 oder 5 Methylengruppen,
         von denen eine CH₂-Gruppe durch Sauerstoff, S, NH, N-CH₃ oder N-Benzyl ersetzt sein kann; wobei
   R(70) wie R(68) oder Amidin;
sowie deren pharmazeutisch verträgliche Salze,
wobei jedoch Verbindungen ausgenommen sind, in denen die Reste R(1) bis R(10) folgendermaßen kombiniert sind:

| R(1) | R(2) | R(3) | R(4) | R(5) | R(6) | R(7) | R(8) | R(9) | R(10) |
|---|---|---|---|---|---|---|---|---|---|
| H | Cl | Cl | H | H | H | H | Cl | Cl | H |
| H | H | NH₂ | H | H | H | H | NH₂ | H | H |
| H | H | H | H | H | H | H | H | H | H |
| Cl | H | H | H | H | H | H | H | H | Cl |
| H | H | Cl | H | H | H | H | Cl | H | H |
| H | H | CH₃ | H | H | H | H | CH₃ | H | H |
| H | H | NH₂ | H | H | H | H | H | H | H |
| H | H | Cl | H | H | H | H | H | H | H |
| H | H | CH₃ | H | H | H | H | H | H | H |

[siehe dazu (von oben an durchgezählt)
Nr. 1: J. Med. Chem. Bd. 6 [1963], Seite 587 (592)
Nr. 2 + 3: Academie des Sciences [1950], S. 1084
Nr. 4, 5, 6, 8 + 9: Bull. Soc. Chim. France [1950], 1038
Nr. 7. C. R. Hebd. Sciences, Acad. Sci [1950], Bd. 230, S 1085.]

Bevorzugt sind Verbindungen der Formel I, in der bedeuten:
R(5) und R(6)
   Wasserstoff;
die übrigen Reste wie in Anspruch 1 definiert sind
   und
R(1) und R(10) gleich sind,
R(2) und R(9) gleich sind,
R(3) und R(8) gleich sind,
R(4) und R(7) gleich sind,
   sowie deren pharmazeutisch verträgliche Salze.

Besonders bevorzugt sind Verbindungen der Formel I, in denen bedeuten:
R(1) und R(10)
   gleich sind und Wasserstoff, F, Cl, CH₃, OH, NH₂ oder CF₃;
R(2) und R(9)
   gleich sind und Wasserstoff, F, Cl, Br, -C≡N, -C_{q}F_{2q}CF₃, R(13)-SO₂, R(14)R(15)N-CO-, R(16)-CO- oder R(17)R(18)N-SO₂-;
   q Null, 1, 2, 3, 4 oder 5;
   R(13), R(14), R(16) und R(17)
      unabhängig voneinander (C₁-C₈)-Alkyl, (C₃-C₄)-Alkenyl, -CₙH₂ₙ-R(20) oder CF₃;
      n Null oder 1;
      R(20) (C₃-C₆)-Cycloalkyl oder Phenyl,
         welches nicht substituiert ist oder substituiert mit 1 - 2 Substituenten aus der Gruppe F, Cl, CF₃, Methyl, Methoxy und NR(21)R(22);
         R(21) und R(22)
            Wasserstoff oder Methyl;
   oder R(14), R(16) und R(17)
      Wasserstoff;
   R(15) und R(18)
      unabhängig voneinander Wasserstoff oder Methyl;
R(3) und R(8)
   gleich sind und -Y-(p-C₆H₄)-(CO)ₕ-(CHOH)ᵢ-(CH₂)ⱼ-(CHOH)ₖ-R(23), -Y-(m-C₆H₄)-(CO)_{ad}-(CHOH)ₐₑ-(CH₂)_{af}-(CHOH)_{ag}-R(24) oder -Y-(o-C₆H₄)-(CO)ₐₕ-(CHOH)ₐₒ-(CH₂)ₐₚ-(CHOH)ₐₖ-R(25);
   Y Sauerstoff, S oder -NR(83);
   R(23), R(24), R(25) und R(83)
      unabhängig voneinander Wasserstoff oder Methyl;
   h, ad und ah
      unabhängig voneinander Null oder 1;
   i, k, ae, ag, ao und ak
      unabhängig voneinander Null, 1, 2 oder 3;
   j, af und ap
      unabhängig voneinander Null oder 1;
      wobei jedoch jeweils h, i und k nicht gleichzeitig Null sind,
      ad, ae und ag nicht gleichzeitig Null sind und
      ah, ao und ak nicht gleichzeitig Null sind,
   oder
R(3) und R(8)
   gleich sind und Wasserstoff, F, Cl, Br, CN, (C₁-C₈)-Alkyl, CF₃, (C₃-C₈)-Alkenyl oder -C_{g}H_{2g}R(26);
   g Null, 1 oder 2;
   R(26) (C₃-C₆)-Cycloalkyl oder Phenyl,
      welches nicht substituiert oder substituiert ist mit 1 - 2 Substituenten aus der Gruppe F, Cl, CF₃, Methyl, Methoxy und NR(27)R(28);
      R(27) und R(28)
         H oder CH₃;
   oder
R(3) und R(8)
   gleich sind und Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl;
      die über C oder N verknüpft sind und die unsubstituiert oder substituiert sind mit 1 - 2 Substituenten aus der Gruppe F, Cl, CF₃, CH₃, Methoxy, Hydroxy, Amino, Methylamino und Dimethylamino;
   oder
R(3) und R(8)
   gleich sind und -W-(p-C₆H₄)-R(96), -W-(m-C₆H₄)-R(97) oder -W-(o-C₆H₄)-R(98);
   R(96), R(97 und R(98)
      unabhängig voneinander Pyrrolyl, Imidazolyl, Pyrazolyl oder Pyridyl,
         das jeweils unsubstituiert oder substituiert ist mit 1 bis 2 Resten aus der Reihe F, Cl, CF₃, CH₃, Methoxy, Dimethylamino und Benzyl;
   W Sauerstoff, -S- oder NR(99)-;
      R(99) Wasserstoff oder Methyl;
   oder
R(3) und R(8)
   gleich sind und -SR(29), -OR(30), -NR(31)R(32) oder -CR(33)R(34)R(35);
   R(29), R(30), R(31) und R(33)
      unabhängig voneinander -CₐH₂ₐ-[Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl],
         die unsubstituiert oder substituiert sind mit 1 - 2 Substituenten aus der Gruppe F, Cl, CF₃, CH₃, Methoxy, Hydroxy, Amino, Methylamino und Dimethylamino;
         a Null oder 1;
   R(32), R(34) und R(35)
      unabhängig voneinander Wasserstoff oder CH₃;
   oder
R(3) und R(8)
   gleich sind und R(72)-SO₂ oder R(73)R(74)N-SO₂-;
   R(72) (C₁-C₄)-Alkyl, CF₃, (C₃-C₄)-Alkenyl oder -CₛH₂ₛ-R(75);
      s Null oder 1;
      R(75) (C₃-C₆)-Cycloalkyl oder Phenyl,
         welches nicht substituiert oder substituiert ist mit 1 - 2 Substituenten aus der Gruppe F, Cl, CF₃, Methyl, Methoxy und NR(76)R(77);
         R(76) und R(77)
            Wasserstoff oder CH₃,
   R(73) Wasserstoff, (C₁-C₄)-Alkyl, CF₃, (C₃-C₄)-Alkenyl oder -C_{w}H_{2w}-R(78);
      w Null oder 1;
      R(78) (C₃-C₆)-Cycloalkyl oder Phenyl,
         welches nicht substituiert oder substituiert ist mit 1 - 2 Substituenten aus der Gruppe F, Cl, CF₃, Methyl, Methoxy und NR(79)R(80);
         R(79) und R(80)
            Wasserstoff oder CH₃;
   R(74) Wasserstoff oder CH₃;
      oder
   R(73) und R(74)
      gemeinsam 4 oder 5 Methylengruppen,
         von denen eine CH₂-Gruppe durch Sauerstoff, S, NH, N-CH₃ oder N-Benzyl ersetzt sein kann;
   oder
R(3) und R(8)
   gleich sind und R(39)X-;
   X Sauerstoff, S, NR(40), (C = O)A- oder NR(41)C = MN⁽*⁾R(42)-,
      M Sauerstoff;
      A Sauerstoff oder NR(43);
         R(43) Wasserstoff, (C₁-C₄)-Alkyl oder (C₁-C₄)-Perfluoralkyl;
      R(41) Wasserstoff, (C₁-C₄)-Alkyl,
      R(42) definiert wie R(39);
   R(39) (C₁-C₆)-Alkyl, (C₃-C₄)-Alkenyl, (CH₂)_{b}C_{d}F_{2d + 1} oder -CₓH₂ₓ-R(44);
      b Null oder 1;
      d 1 - 7;
      x Null oder 1,
      R(44) (C₃-C₆)-Cykloalkyl oder Phenyl,
         welches nicht substituiert oder substituiert ist mit 1 - 2 Substituenten aus der Gruppe F, Cl, CF₃, Methyl, Methoxy und NR(45)R(46);
         R(45) und R(46)
            Wasserstoff oder CH₃;
      oder
   R(39) und R(40) beziehungsweise R(39) und R(41)
      gemeinsam 4 oder 5 Methylengruppen,
         von denen eine CH₂-Gruppe durch Sauerstoff, S, NH, N-CH₃ oder N-Benzyl ersetzt sein kann;
      wobei A und N⁽*⁾ an den Phenylkern des Benzoylguanidin-Grundkörpers gebunden sind;
   oder
R(3) und R(8)
   gleich sind und -SR(47), -OR(48), -NHR(49), -NR(50)R(51), -CHR(52)R(53), -CR(54)R(55)OH, -C≡C-R(56), -CR(58)=CHR(57) oder -[CR(59)R(60)]ᵤ-CO-[CR(61)R(62)]ᵥ-R(63);
   R(47), R(48), R(49), R(50) und R(52)
      gleich oder verschieden -(CH₂)_{y}-(CHOH)_{z}-(CH₂)ₐₐ-(CHOH)ₜ-R(64) oder -(CH₂)_{ab}-O-(CH₂-CH₂O)_{ac}-R(65);
      y, z, aa, ab, ac
         gleich oder verschieden Null, 1 oder 2;
      R(64) und R(65)
         Wasserstoff oder Methyl;
   u 1 oder 2;
   v Null, 1 oder 2;
      t 1, 2 oder 3;
   R(51), R(53), R(54) und R(55)
      gleich oder verschieden Wasserstoff oder Methyl;
      oder
   R(52) und R(53) beziehungsweise R(54) und R(55)
      zusammen mit dem sie tragenden Kohlenstoff-Atom ein (C₃-C₆)-Cycloalkyl,
   R(63) Wasserstoff, (C₁-C₄)-Alkyl, (C₃-C₆)-Cycloalkyl oder -CₑH₂ₑ-R(81);
      e Null, 1 oder 2;
   R(56), R(57), R(81)
      unabhängig voneinander Phenyl,
         das unsubstituiert oder substituiert ist mit 1 - 2 Substituenten aus der Gruppe F, Cl, CF₃, Methyl, Methoxy oder NR(65)R(66) mit R(65) und R(66) gleich Wasserstoff oder CH₃;
      oder
   R(56), R(57) und R(81)
      unabhängig voneinander Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl,
         die unsubstituiert oder wie Phenyl substituiert sind;
   R(58), R(59), R(60), R(61) und R(62)
      Wasserstoff, Methyl;
   oder
R(3) und R(8)
   R(67)-NH-SO₂-;
   R(67) R(68)R(69)N-(C = Y')-;
      Y' Sauerstoff, S oder N-R(70);
      R(68) und R(69)
         gleich oder verschieden Wasserstoff, (C₁-C₄)-Alkyl, (C₃-C₄)-Alkenyl oder -C_{f}H_{2f}-R(71);
         f Null oder 1;
         R(71) (C₅-C₇)-Cycloalkyl oder Phenyl,
            welches unsubstituiert oder substituiert ist mit 1 - 2 Substituenten aus der Gruppe F, Cl, CF₃, Methoxy und Methyl;
         oder
      R(68) und R(69)
         gemeinsam 4 oder 5 Methylengruppen,
            von denen eine CH₂-Gruppe durch Sauerstoff, S, NH, N-CH₃ oder N-Benzyl ersetzt sein kann,
         R(70) wie R(68) definiert ist;
R(4) und R(7)
   gleich sind und (C₁-C₈)-Alkyl, -CₐₗH₂ₐₗR(84) oder CF₃;
   al Null oder 1;
   R(84) (C₃-C₆)-Cycloalkyl oder Phenyl,
      welches nicht substituiert oder substituiert ist mit 1 - 2 Substituenten aus der Gruppe F, Cl, CF₃, Methyl, Methoxy und NR(85)R(86);
      R(85) und R(86)
         Wasserstoff oder CH₃;
   oder
R(4) und R(7)
   gleich sind und Chinolyl, Isochinolyl, Pyrrolyl, Pyridyl oder Imidazolyl,
      die über C oder N verknüpft sind und die unsubstituiert oder substituiert sind mit 1 - 2 Substituenten aus der Gruppe F, Cl, CF₃, CH₃, Methoxy, Hydroxy, Amino, Methylamino und Dimethylamino;
   oder
R(4) und R(7)
   gleich sind und R(87)-SOₐₘ oder R(88)R(89)N-SO₂-;
   am 2;
   R(87) (C₁-C₄)-Alkyl oder CF₃;
   R(88) Wasserstoff, (C₁-C₄)-Alkyl, CF₃ oder -CₐₙH₂ₐₙ-R(90),
      an Null oder 1;
      R(90) Phenyl,
         welches nicht substituiert oder substituiert ist mit 1 - 2 Substituenten aus der Gruppe F, Cl, CF₃, Methyl, Methoxy und NR(90)R(91);
         R(90) und R(91)
            Wasserstoff oder CH₃;
   R(89) Wasserstoff oder CH₃;
   oder
R(4) und R(7)
   gleich sind und -C≡CR(93);
   R(93) Phenyl,
      das unsubstituiert oder substituiert ist mit 1 - 2 Substituenten aus der Gruppe F, Cl, CF₃, Methyl, Methoxy und NR(94)R(95);
      R(94) und R(95)
         Wasserstoff oder CH₃;
R(5) und R(6)
   gleich sind und Wasserstoff;
   sowie deren pharmazeutisch verträgliche Salze.

Aus der WO 84/875 sind Guanidin-Verbindungen bekannt, die jedoch zwischen der Phenylgruppe und der Carbonylgruppe stets eine Alkylenbrücke tragen, da deren Substituent R(1) nie ein Phenyl, sondern im nächstkommenden Fall ein Phenylalkyl- oder ein Phenylalkenylrest ist; es handelt sich also nicht um Dibenzoylguanidine.

Unter (C₁-C₉)-Heteroaryl werden insbesondere Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Des weiteren können auch ein oder beide Atome der Kondensationsstelle bicyclischer Reste (wie im Indolizinyl) N-Atome sein.

Als Heteroaryl gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl.

Enthält einer der Substituenten R(1) bis R(10) ein oder mehrere Asymmetriezentren, so können diese sowohl S als auch R konfiguriert sein. Die Verbindungen können als optische Isomere, als Diastereomere, als Racemate oder als Gemische derselben vorliegen.

Die bezeichneten Alkyl- und Perfluoralkyl-reste können sowohl geradkettig wie verzweigt vorliegen.

Die Erfindung betrifft weiterhin ein Verfahren zur Herstellung der Verbindungen I, dadurch gekennzeichnet, daß man
a) je ein Äquivalent der Verbindungen der Formel II aa und II bb mit einem Äquivalent Guanidin umsetzt, 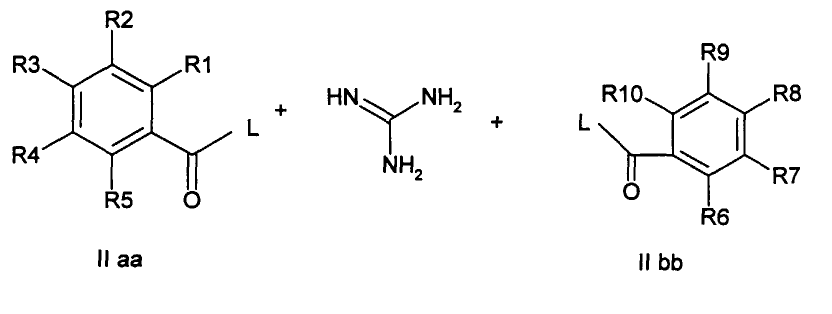 worin R(1) bis R(10) die angegebene Bedeutung besitzen und L für eine leicht nucleophil substituierbare leaving group steht, oder
b) eine Verbindung der Formel II aa 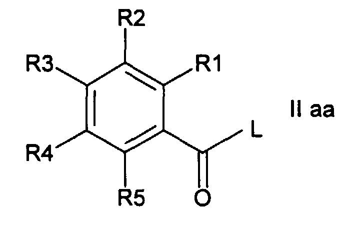 mit einer Verbindung der Formel III aa 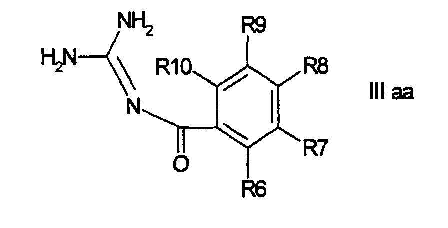 unter Beteiligung einer Base umsetzt, worin R(1) bis R(10) die angegebenen Bedeutungen besitzen und L für eine leicht nucleophil substituierbare leaving group steht,
   und daß man gegebenenfalls in ein pharmazeutisch verträgliches Salz überführt.

Die aktivierten Säurederivate der Formel II a und II b, worin L eine Alkoxy-, vorzugsweise eine Methoxygruppe, eine Phenoxygruppe, Phenylthio, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.

Neben den Carbonsäurechloriden der Formel II (L = Cl) lassen sich auch weitere aktivierte Säurederivate der Formel II in an sich bekannter Weise direkt aus den zugrundeliegenden Benzoesäure-derivaten (Formel II, L = OH) herstellen, wie beispielsweise die Methylester der Formel II mit L = OCH₃ durch Behandeln mit gasförmigem HCl in Methanol, die Imidazolide der Formel II durch Behandeln mit Carbonyldiimidazol [L = 1-Imidazolyl, Staab, Angew. Chem. Int. Ed. Engl. 1, 351 - 367 (1962)], die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit O-[(Cyano(ethoxycarbonyl)methylen)amino]-1,1,3,3-tetramethyluronium-tetrafluorborat ("TOTU") [Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäure-derivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in an sich bekannter Weise in einem protischen oder aprotischen polaren aber inerten organischen Lösungsmittel. Dabei haben sich bei der Umsetzung der Benzoesäuremethylester (II, L = OMe) mit Guanidin Methanol, Isopropanol oder THF von 20°C bis zur Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II mit Guanidin verwendet werden.

Wenn L = Cl bedeutet, arbeitet man vorteilhaft unter Zusatz eines Säurefängers, z. B. in Form von überschüssigen Guanidin zur Abbindung der Halogenwasserstoffsäure.

Ein Teil der zugrundeliegenden Benzoesäurederivate der Formel II ist bekannt und in der Literatur beschrieben. Die unbekannten Verbindungen der Formel II können nach literaturbekannten Methoden hergestellt werden. Die Einführung einiger Substituenten gelingt durch literaturbekannte Methoden des Palladiumvermittelten cross-couplings von Arylhalogeniden bzw. Aryltriflaten mit beispielsweise Organostannanen, Organoboronsäuren, Organoboranen, Organokupfer- bzw. Organozink-Verbindungen oder terminalen Alkinen. Die erhaltenen Benzoesäuren werden nach einer der oben beschriebenen Verfahrensvarianten zu aktivierten Benzoesäurederivaten der Formel II umgesetzt. Die Verbindungen der Formel II werden entweder direkt zu den erfindungsgemäßen Verbindungen der Formel I umgesetzt, oder zunächst mit Guanidin zu Verbindungen der Formel III überführt, die nach Isolierung durch Umsetzung mit einer weiteren Verbindung der Formel II zu den erfindungsgemäßen Verbindungen der Formel I umgesetzt werden.

Dialkanoylsubstituierte Guanidine der Formel I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind dialkanoylsubstituierte Guanidine, die sowohl direkt den zellulären Natrium-Protonen-Austauscher ( Na ⁺ /H ⁺ -exchanger oder Na ⁺ /H ⁺ -antiporter) inhibieren, als auch in vivo mit einer Halbwertszeit zwischen 1 Minute und 10 Stunden einen ihrer Acylreste verlieren und so wiederum Monoacylguanidine, wirkungsvolle Inhibitoren des zellulären Natrium-Protonen-Austauscher, freisetzen. Damit sind Verbindungen der Formel I potente Inhibitoren des zellulären Natrium-Protonen-Austauscher und führen zu einer Verbesserung der Kinetik der zugrundeliegenden Monoacylguanidine. Darüber hinaus führen sie aufgrund ihrer Lipophilie zu gegenüber den Monoacylguanidinen erhöhten ZNS-Konzentration an Wirkstoff in Form von Dialkanoyl- und Monoacylguanidinen.

Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na ⁺ /H ⁺ -Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüberhinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüberhinaus zeichnen sich die erfindungsgemäßen Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na ⁺ /H ⁺ -Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg,
vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

### Liste der Abkürzungen:

- Bn: Benzyl
- CH₂Cl₂: Dichlormethan
- DCI: Desorption-Chemical Ionisation
- DIP: Diisopropylether
- DME: Dimethoxyethan
- DMF: N,N-Dimethylformamid
- EE: Ethylacetat (EtOAc)
- EI: electron impact
- eq.: Äquivalent
- ES: Elektrospray-Ionisation
- Et: Ethyl
- FAB: Fast Atom Bombardment
- HEP: n-Heptan
- Me: Methyl
- MeOH: Methanol
- mp: Schmelzpunkt
- MTB: Methyltertiärbutylether
- Pd/C: Palladium auf Kohle
- Pt/C: Platin auf Kohle
- RT: Raumtemperatur
- THF: Tetrahydrofuran
- ZNS: Zentralnervensystem

### Experimenteller Teil

### Allgemeine Vorschrift zur Herstellung von Monoacyl-guanidinen (III)

### Variante A: aus Carbonsäuren (II, L = OH)

1.0 eq. des Carbonsäurederivates der Formel II löst bzw. suspendiert man in wasserfreiem THF ( 5 ml/mmol ) und versetzt sodann mit 1.1 eq. Carbonyldiimidazol. Nach dem Rühren über 2 Stunden bei RT werden 5.0 eq. Guanidin in die Reaktionslösung eingetragen. Nach dem Rühren über Nacht destilliert man das THF unter vermindertem Druck im Rotationsverdampfer ab, versetzt mit Wasser, stellt mit 2N HCL auf pH 6 bis 7 und filtriert das entsprechende Monoacyl-guanidin (Formel III) ab. Die so erhaltenen Monoacyl-guanidine können durch Behandeln mit wäßriger, methanolischer oder etherischer Salzsäure oder anderen pharmakologisch verträglichen Säuren in die entsprechenden Salze übergeführt werden.

### Allgemeine Vorschrift zur Herstellung von Monoacyl-guanidinen (III)

### Variante B: aus Carbonsäure-alkylestern (II, L = O-Alkyl)

1.0 eq. des Carbonsäure-alkylesters der Formel II sowie 5.0 eq. Guanidin (freie Base) werden in Isopropanol gelöst oder in THF suspendiert und bis zum vollständigen Umsatz (Dünnschichtkontrolle) unter Rückfluß gekocht (typische Reaktionszeit 2 bis 5 h). Das Lösungsmittel wird unter vermindertem Druck im Rotationsverdampfer abdestilliert, dann wird in EE aufgenommen und 3 x mit NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert.
(Salzbildung vergleiche Variante A)

### Allgemeine Vorschrift zur Darstellung von Diacylguanidinen (I)

### Variante F: aus Carbonsäuren (II, L = OH)

2.0 eq. des Carbonsäurederivates der Formel II löst bzw. suspendiert man in wasserfreiem THF ( 5 ml/mmol ) und versetzt sodann mit 2.2 eq. Carbonyldiimidazol. Nach dem Rühren über 2 Stunden bei RT werden 1.0 eq. Guanidin in die Reaktionslösung eingetragen. Nach dem Rühren über Nacht destilliert man das THF unter vermindertem Druck im Rotationsverdampfer ab, versetzt mit Wasser, stellt mit 2N HCl auf pH 6 bis 7 und filtriert das entsprechende Dialkanoyl-guanidin (Formel I) ab. Die so erhaltenen Monoacyl-guanidine können durch Behandeln mit wäßriger, methanolischer oder etherischer Salzsäure oder anderen pharmakologisch verträglichen Säuren in die entsprechenden Salze überführt werden.

### Allgemeine Vorschrift zur Darstellung von Diacylguanidinen (I)

### Variante G: aus Carbonsäureestern (II, L = O-Alkyl)

2.0 eq. des Carbonsäure-alkylesters der Formel II sowie 1.0 eq. Guanidin (freie Base) werden in Isopropanol gelöst oder in THF suspendiert und bis zum vollständigen Umsatz (Dünnschichtkontrolle) unter Rückfluß gekocht (typische Reaktionszeit 2 bis 5 h). Das Lösungsmittel wird unter vermindertem Druck im Rotationsverdampfer abdestilliert in EE aufgenommen und 3 x mit NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert.
(Salzbildung vergleiche Variante A)

### Allgemeine Vorschrift zur Herstellung von Diacyl-guanidinen (I)

### Variante K: aus Monoacyl-guanidinen (III) und einem Carbonsäurederivat der Formel II ( zum Beispiel Carbonsäureester oder aktivierte Carbonsäure)

1.0 eq. des Monoacyl-guanidins der Formel III ( freie Base ) sowie 1.0 eq. des Carbonsäurederivats der Formel II ( zur Darstellung einer aktivierten Carbonsäure vergleiche Variante A) werden in Isopropanol oder in THF gelöst bzw. suspendiert und bis zum vollständigen Umsatz (Dünnschichtkontrolle) bei passender Temperatur (RT bis Rückfluß) gerührt. Das Lösungsmittel wird unter vermindertem Druck im Rotationsverdampfer abdestilliert in EE aufgenommen und 3 x mit NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 10 : 1 chromatographiert.
(Salzbildung vergleiche Variante A)

### Beispiel 1

### Bis-(3-Methylsulfonyl-4-i-propyl-benzoyl)-guanidin

### a) 3-Methylsulfonyl-4-i-propyl-benzoylchlorid

4.0 g 3-Methylsulfonyl-4-i-propyl-benzoesäure, 1.5 ml Thionylchlorid und 3 Tropfen DMF werden in 30 ml Toluol 10 h unter Rückfluß erhitzt. Anschließend wird das Solvens im Vakuum entfernt und das Produkt ohne Reinigung weiter eingesetzt.

### b) Bis-(3-Methylsulfonyl-4-i-propyl-benzoyl)-guanidin

Das Säurechlorid 1 a) und 3.9 g 3-Methylsulfonyl-4-i-propyl-benzoylguanidin werden in 50 ml DMF gelöst, und dann werden 3.4 g K₂CO₃ zugegeben. 4 h wird bei RT gerührt und über Nacht stehen gelassen. Die Lösung wird anschließend eingeengt und der Rückstand in 200 ml Wasser verrührt. Der Feststoff wird abfiltriert und mit 100 ml Wasser gewaschen. Nun wird dieser Feststoff in 100 ml EE gelöst und 1 x mit 10 ml 1 N HCl und anschließend 1x mit 50 ml NaCl-Lösung gewaschen. Über Na₂SO₄ wird getrocknet und das Solvens im Vakuum entfernt. Man erhält einen gelben Feststoff , der durch zweimaliges Verreiben mit 20 ml Diethylether und anschließendes Abfiltrieren gereinigt wird. Das Produkt wird im Vakuum getrocknet und man erhält 1.9 g eines weißen Feststoffs, mp 218 - 221 °C.
R_{f} (CH₂Cl₂/MeOH 20 : 1) = 0.57 MS (ES) : 508 (M + 1)

### Vorstufen

4-Isopropyl-3-methylsulfonyl-benzoylguanidin-methansulfonat:
Farblose Kristalle, mp 226 - 28°C,

### Syntheseweg:

a) 4-Isopropyl-benzoesäure durch Oxidation von 4-Isopropyl-benzaldehyd mit Natriumperborat in Essigsäure bei 50°C, mp. 118°C.
b) 4-Isopropyl-3-chlorsulfonyl-benzoesäure aus a) durch Erhitzen in Chlorschwefelsäure bei 95°C für 3 h, mp 203 - 4°C,
c) 2-Isopropyl-5-carboxy-benzolsulfinsäure aus b) durch Reduktion mit Natriumsulfit bei 60°C in wäßriger Natronlauge (pH ^{∼} 9 - 10), mp 205 - 7°C,
d) 4-Isopropyl-3-methylsulfonyl-benzoesäure aus c) durch Alkylierung mit Methylbromid in Gegenwart von NaOH in DMF bei 60°C für 3 h, mp 209 - 11°C,
e) 4-Isopropyl-3-methylsulfonyl-benzoylguanidin-methansulfonat aus d) durch Reaktion mit Thionylchlorid in Toluol (Rückfluß) für 1 h. Nach Abziehen des Toluols wird in THF aufgenommen und das entstandene Säurechlorid zu einem Gemisch aus Guanidin-hydrochlorid, 2N NaOH und THF gegeben. Nach 4 h Rühren bei 30 - 40°C wird das THF abdestilliert, wobei das Produkt als freie Base kristallin anfällt. Anschließende Behandlung mit Methansulfonsäure liefert das Salz.

### Beispiel 2

### Bis-(4-Fluor-3-trifluormethyl-benzoyl)-guanidin

### a) 4-Fluor-3-trifluormethyl-benzoylchlorid

1.5 g 4-Fluor-3-trifluormethyl-benzoesäure, 0.65 ml Thionylchlorid und zwei Tropfen DMF werden in 17 ml Toluol 12 h unter Rückfluß erhitzt, anschließend konzentriert und ohne weitere Reinigung eingesetzt.

### b) Bis-(4-Fluor-3-trifluormethyl-benzoyl)-guanidin

Das Säurechlorid 2a) und 0.87 g 4-Fluor-3-trifluormethyl-benzoylguanidin werden in 15 ml DMF gelöst, und dann werden 1.3 g Kaliumcarbonat zugesetzt. Es wird 20 h bei rt gerührt und wie unter 1 b) beschrieben aufgearbeitet. Das erhaltene, klare Öl wird säulenchromatographisch gereinigt (kieselgel, Heptan/Essigester 8:2) und man erhält einen farblosen Feststoff, mp 143-45°C.
R_{f} (Heptan/Essigester 7:3) = 0.7; MS (ES) : 440 (M + 1)

### Vorstufe

4-Fluor-3-trifluormethyl-benzoylguanidin:
Farblose Kristalle, mp 159-60°C

### Syntheseweg:

4-Fluor-3-trifluormethyl-benzoylguanidin aus 4-Fluor-3-trifluormethyl-benzoesäure durch Reaktion mit N,N*'*-Carbonyldiimidazol in THF bei rt und anschließende Zugabe von Guanidin

### Beispiel 3

### Bis-[4-(1-Imidazolyl)-3-trifluormethyl-benzoyl]-guanidin und

### Beispiel 4

### N-[4-(1-Imidazolyl)-3-trifluormethyl-benzoyl]-N'-(4-Fluor-3-trifluormethylbenzoyl)-guanidin-hydrochlorid

0.25 g Bis-(4-Fluor-3-trifluormethyl-benzoyl)-guanidin, 0.16 g Imidazol und 0.16 g Kaliumcarbonat werden in 5 ml DMF für 16 h erhitzt und danach das Solvens evaporiert. Säulenchromatische Trennung des Rohprodukts ergab 0.12 g Bis-[4-(1-Imidazolyl)-3-trifluormethyl-benzoyl]-guanidin als farblosen Feststoff, mp 130°C, dec., R_{f} (CH₂Cl₂/MeOH 9:1) = 0.4 MS (ES) : 536 (M + 1) und 0.07 g N-[4-(1-Imidazolyl)-3-trifluormethyl-benzoyl]-N*'*-(4-Fluor-3-trifluormethyl-benzoyl)-guanidin als Öl. Behandlung mit HCl_{g}/Ether ergab 0.06 g N-[4-(1-Imidazolyl)-3-trifluormethyl-benzoyl]-N*'*-(4-Fluor-3-trifluormethylbenzoyl)-guanidin-hydrochlorid als farblosen Feststoff, mp 217-218, dec., R_{f} (CH₂Cl₂/MeOH 9:1) = 0.53; MS (ES) : 488(M + 1)

### Pharmakologische Daten:

### Inhibition des Na ⁺ /H ⁺ -Exchangers von Kaninchenerythozyten:

Weiße Neuseeland-Kaninchen (Ivanovas) erhielten eine Standard-Diät mit 2% Cholesterin für sechs Wochen, um den Na ⁺ /H ⁺ -Austausch zu aktivieren und so den Na ⁺ -Influx in die Erythrozyten via Na ⁺ /H ⁺ -Austausch flammenphotometrisch bestimmen zu können. Das Blut wurde den Ohrarterien entnommen und durch 25 lE/ml Kalium-Heparin ungerinnbar gemacht. Ein Teil jeder Probe wurde zur Doppelbestimmung des Hämatokrits durch Zentrifugation benutzt. Aliquots von jeweils 100 µl dienten zur Messung des Na ⁺ -Ausgangsgehalts der Erythrozyten.

Um den Amilorid-sensitiven Natrium-Influx zu bestimmen, wurden 100 µl jeder Blutprobe in jeweils 5 ml eines hyperosmolaren Salz-Sucrose-Mediums (mmol/l: 140 NaCl, 3 KCl, 150 Sucrose, 0,1 Ouabain, 20 Tris-hydroxymethylaminomethan) bei pH 7,4 und 37°C inkubiert. Die Erythrozyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na ⁺ -Nettoinflux wurde aus der Differenz zwischen Natrium-Ausgangswerten und dem Natriumgehalt der Erythrozyten nach Inkubation errechnet. Der Amilorid-hemmbare Natrium-Influx ergab sich aus der Differenz des Natriumgehalts der Erythrozyten nach Inkubation mit und ohne Amilorid 3 x 10⁻⁴ mol/l. Auf diese Weise wurde auch bei den erfindungsgemäßen Verbindungen verfahren.

### Ergebnisse zur Inhibition des Na ⁺ /H ⁺ -Exchangers:

| IC₅₀(µmol) | Beispiel |
|---|---|
| 10 | 1 |
| 10 | 2 |
| 5 | 3 |
| 3 | 4 |

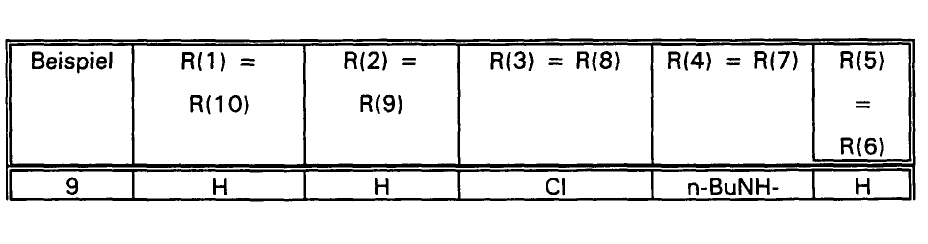

### Analog zu Beispiel 1 werden erhalten:

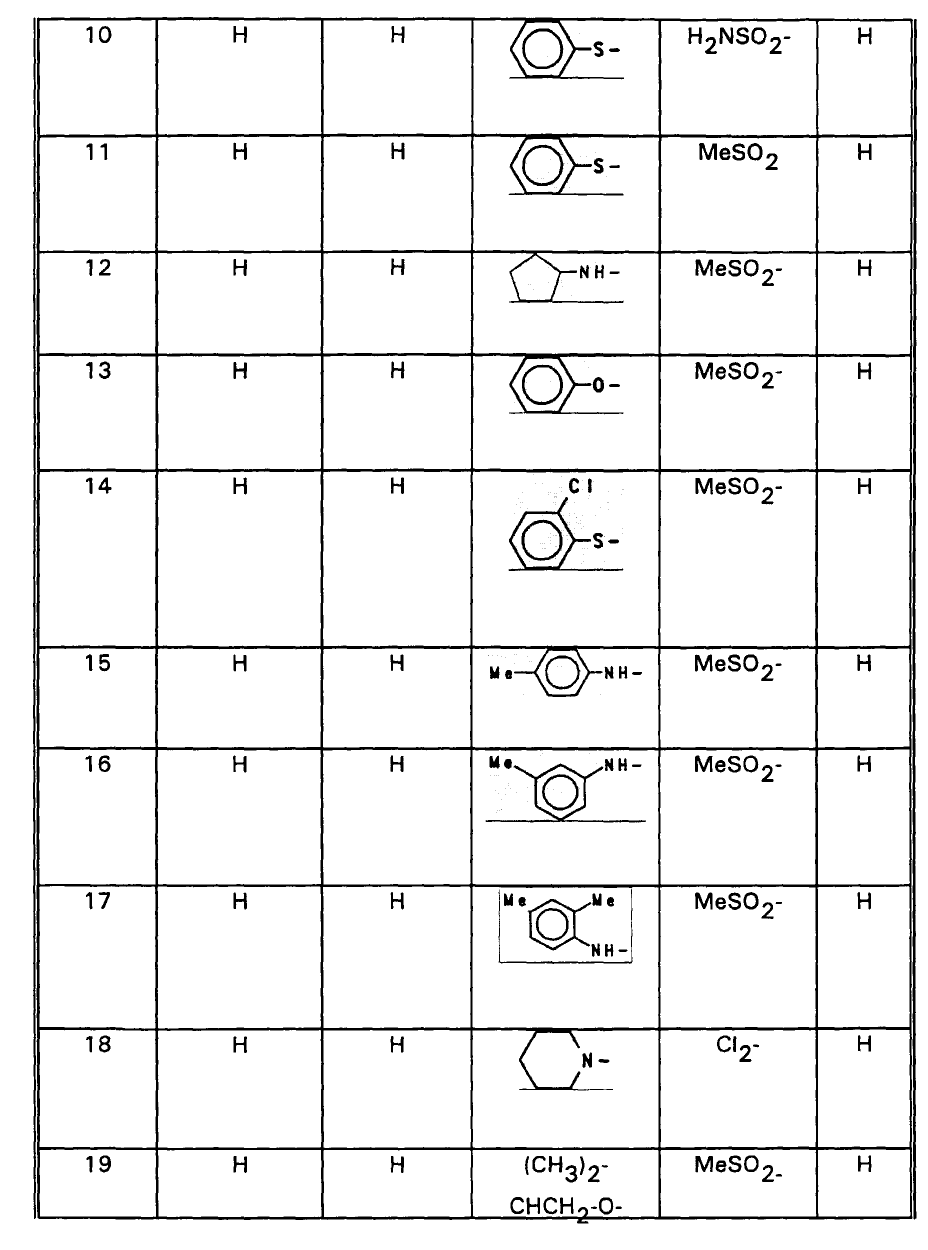

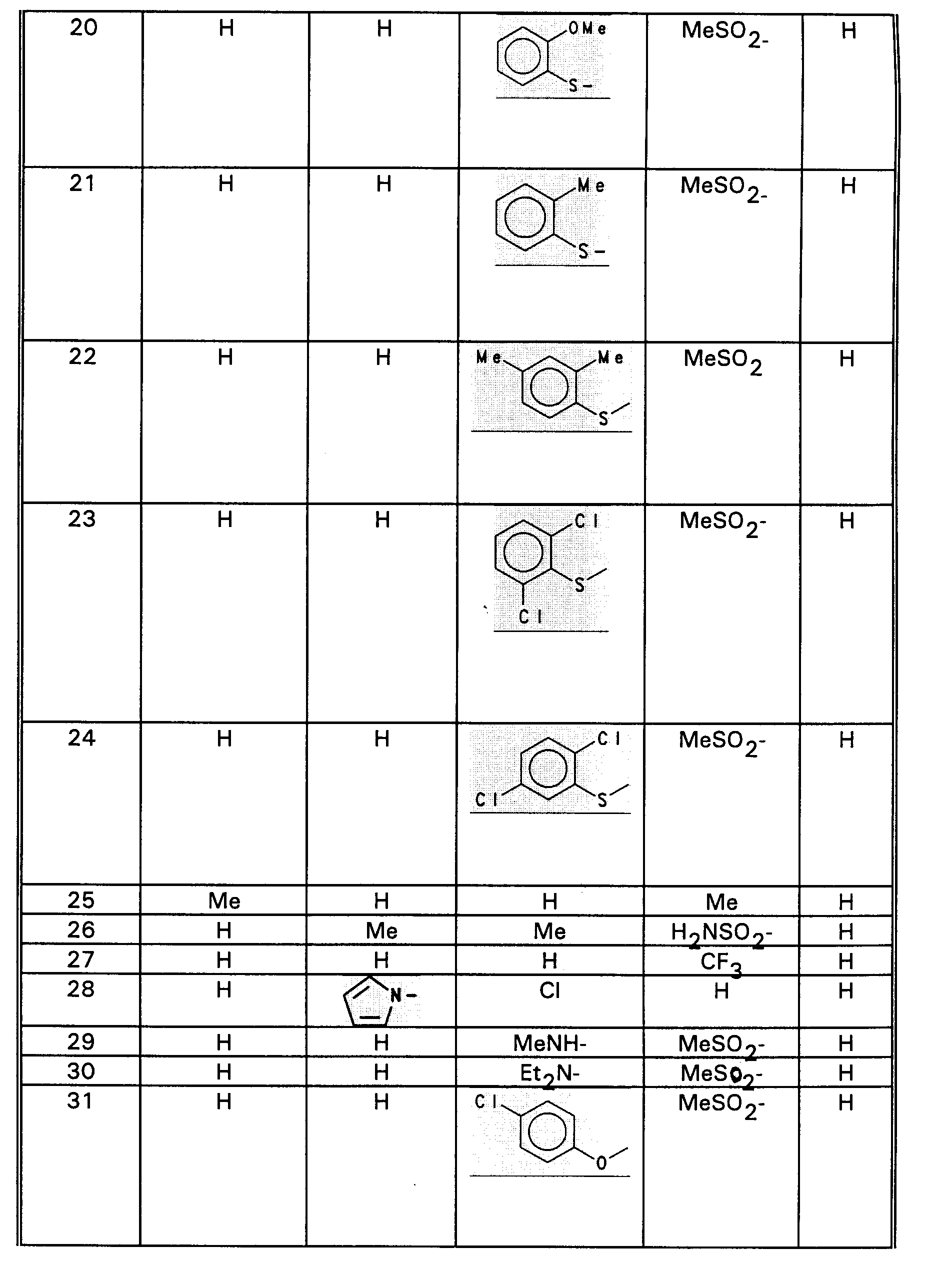

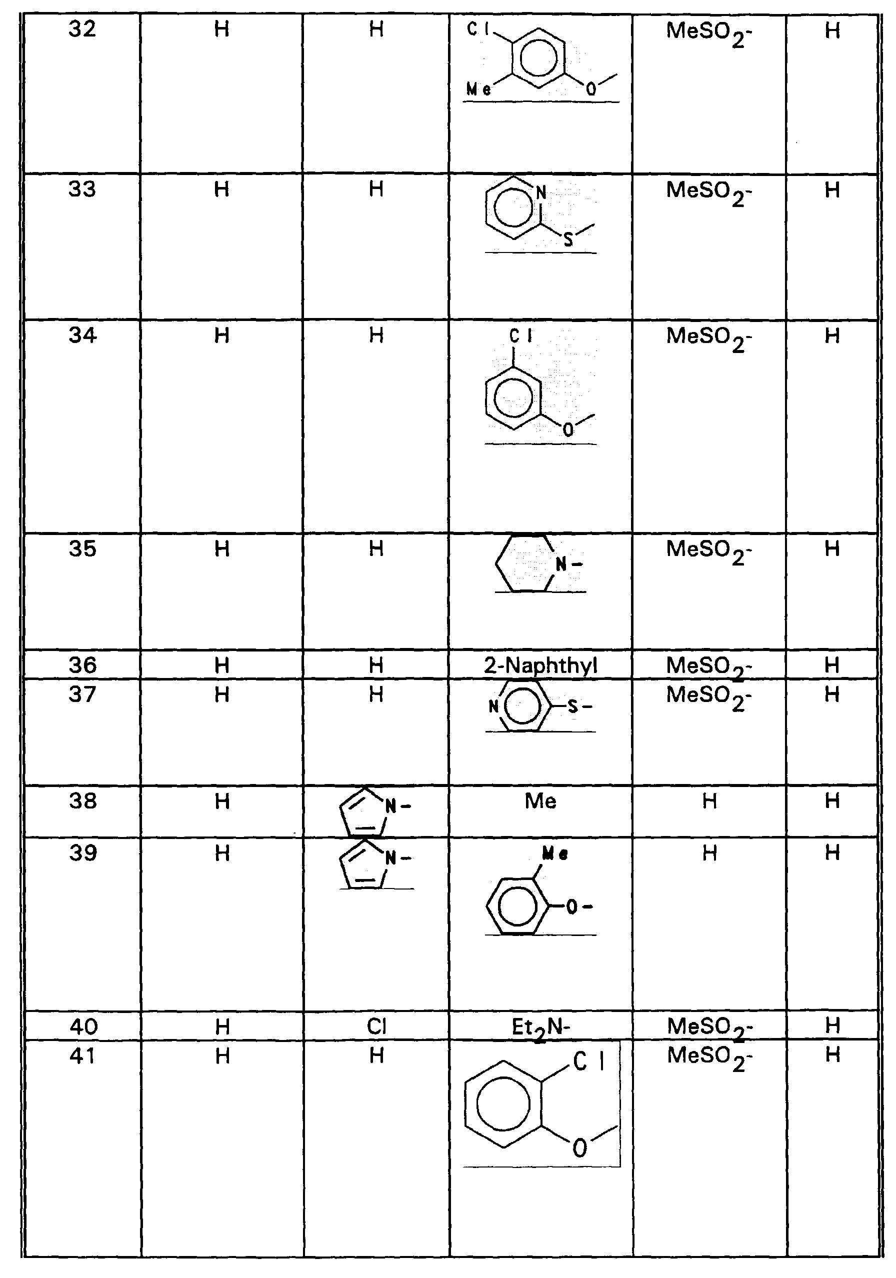

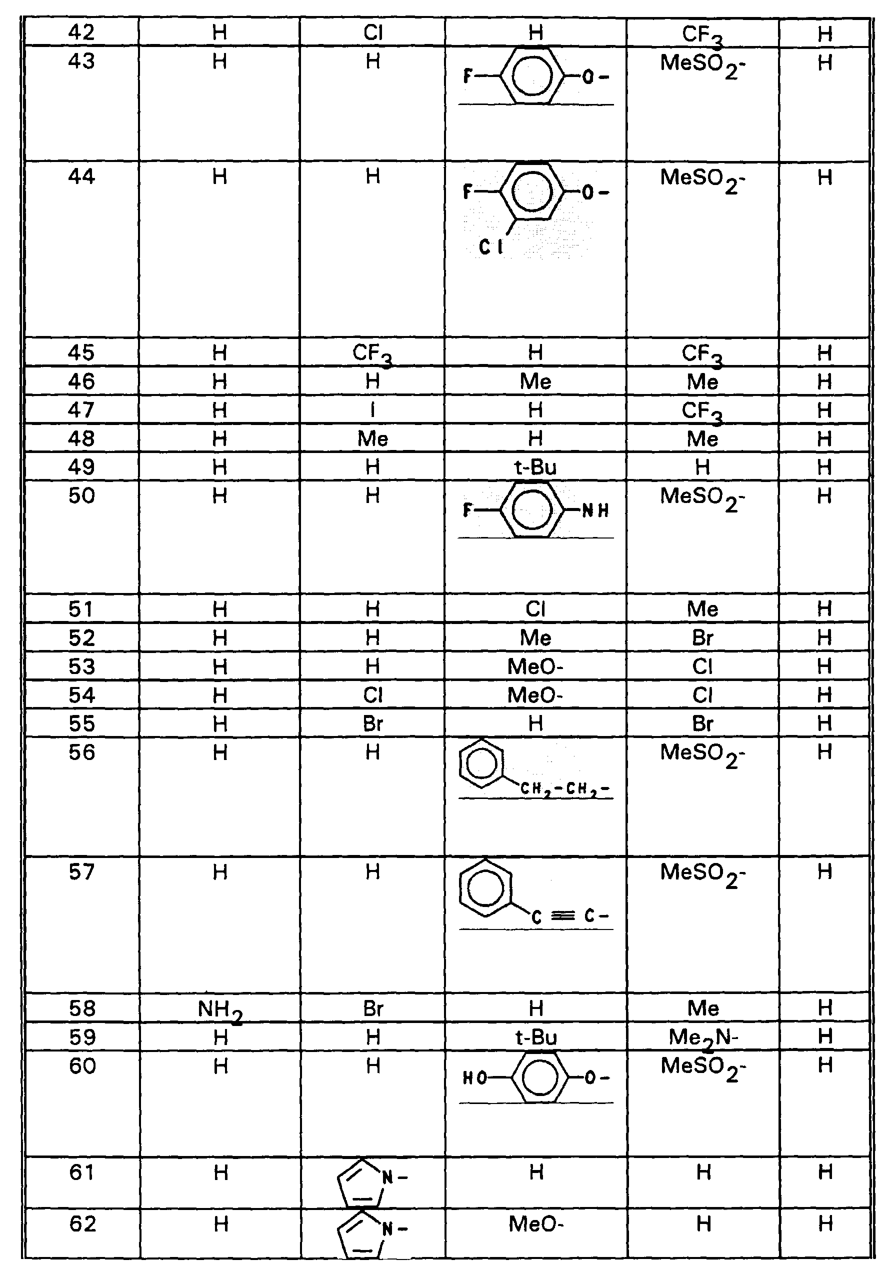

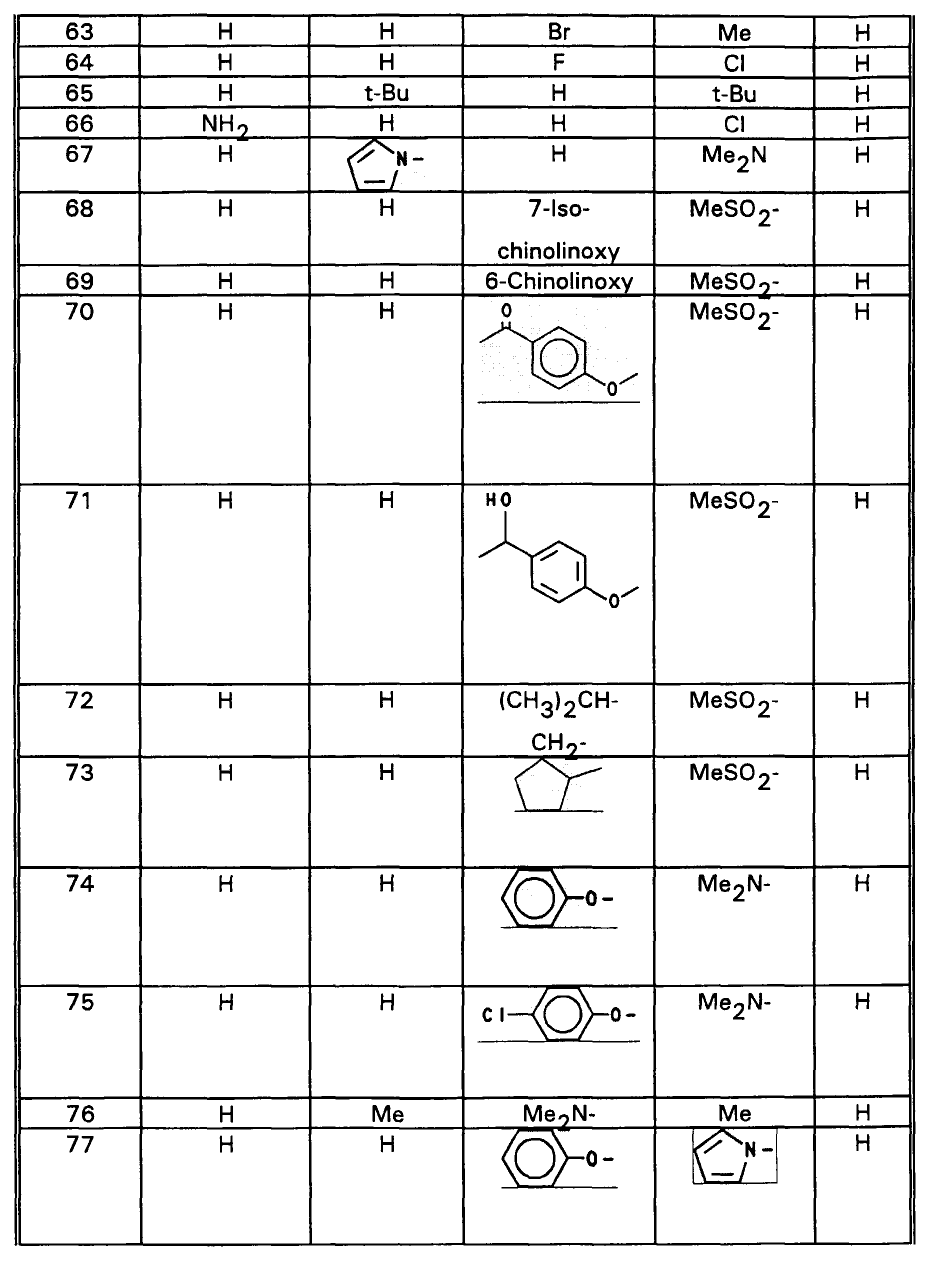

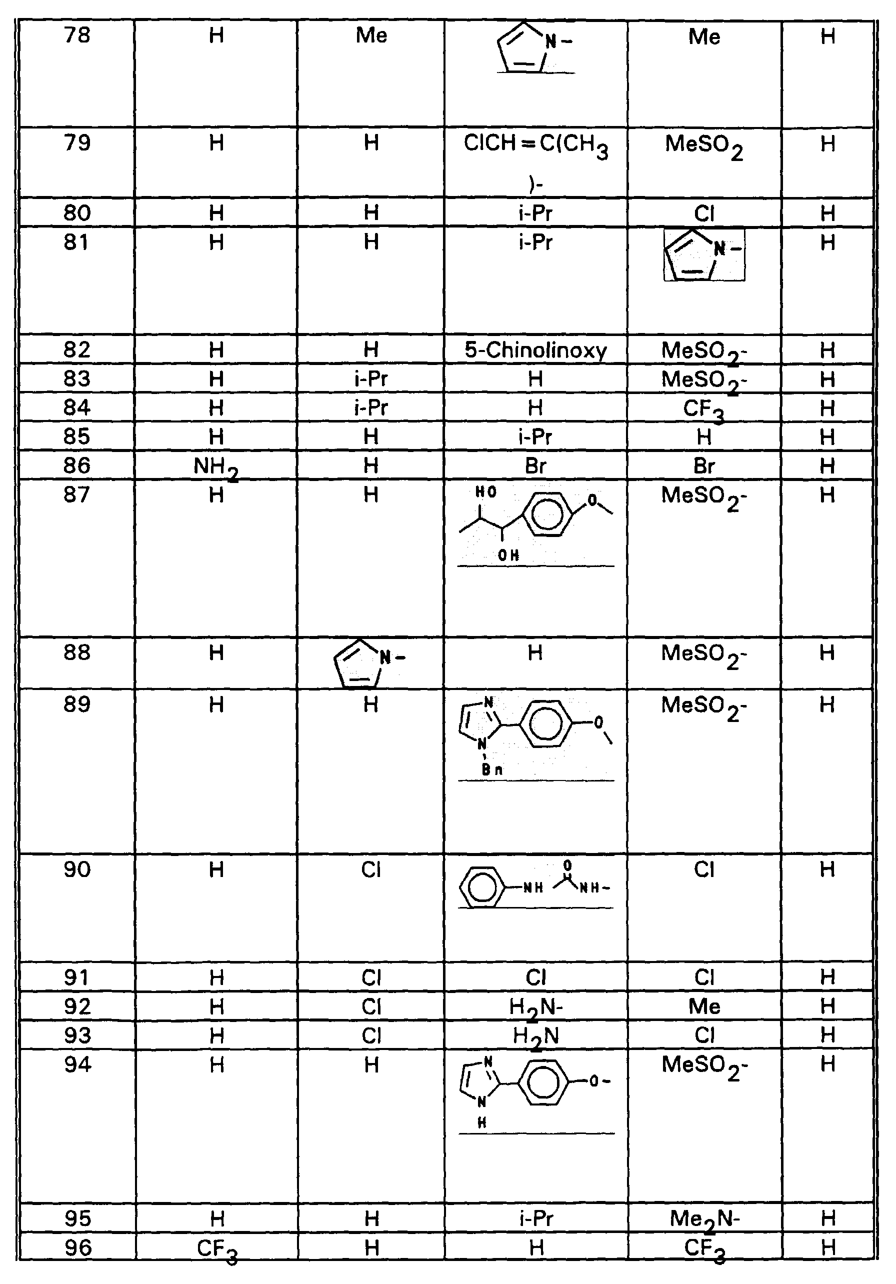

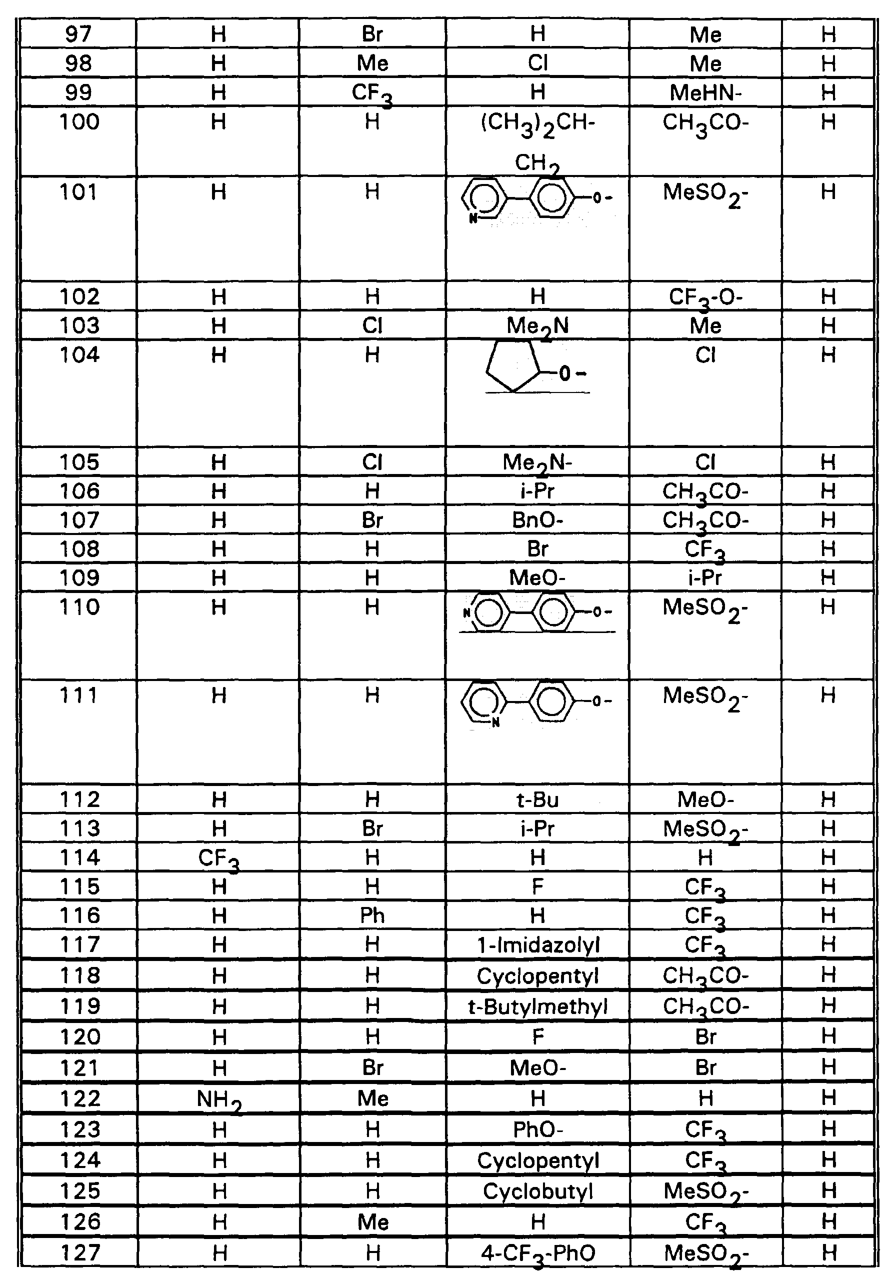

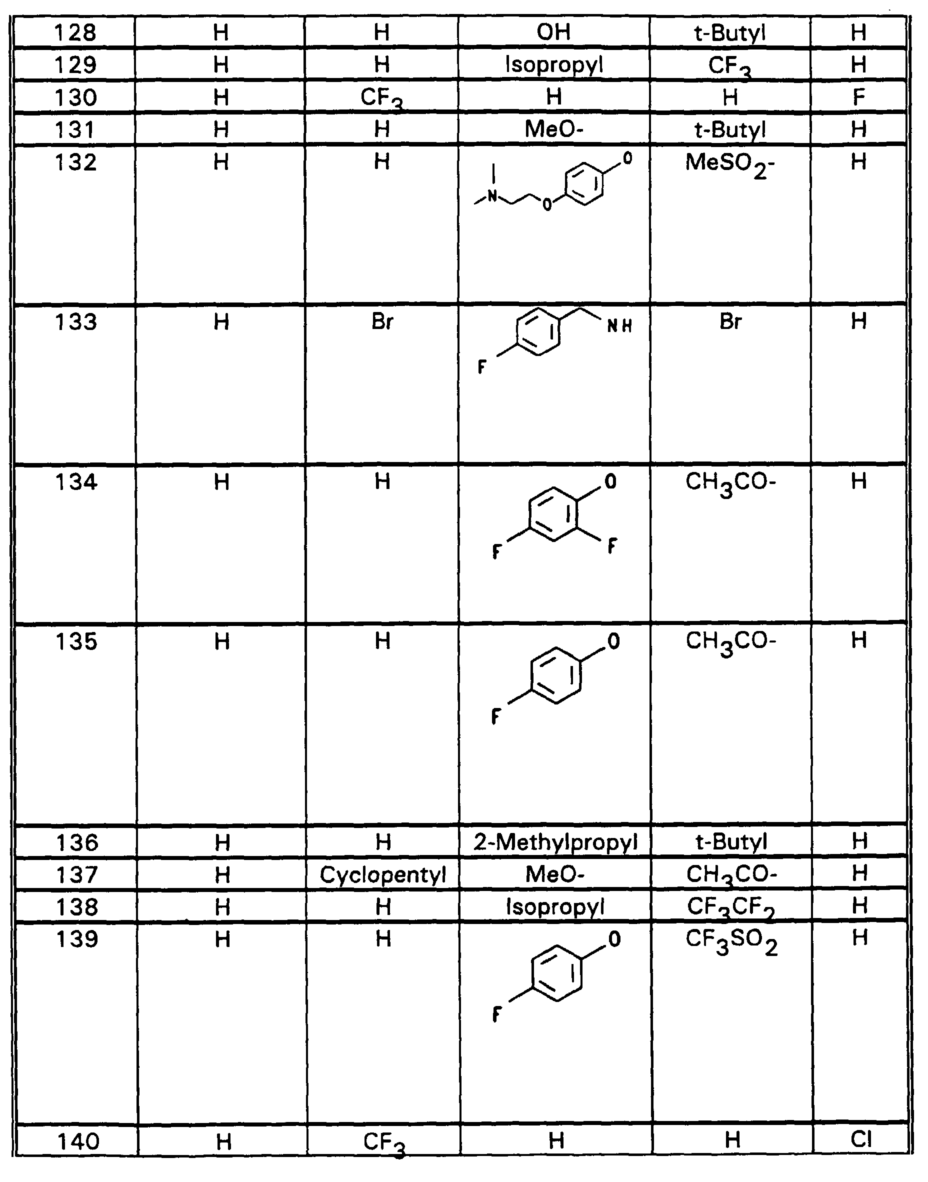

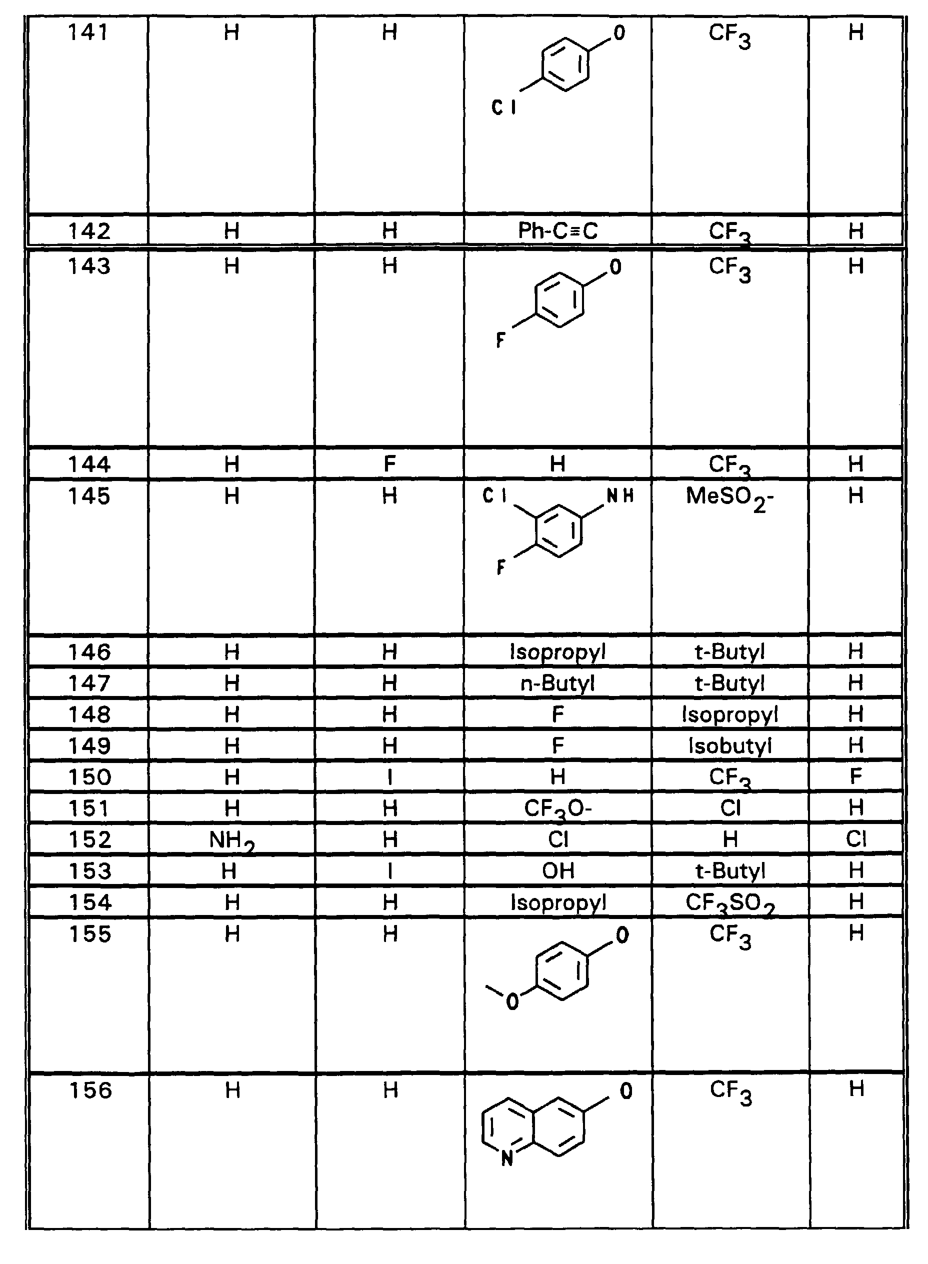

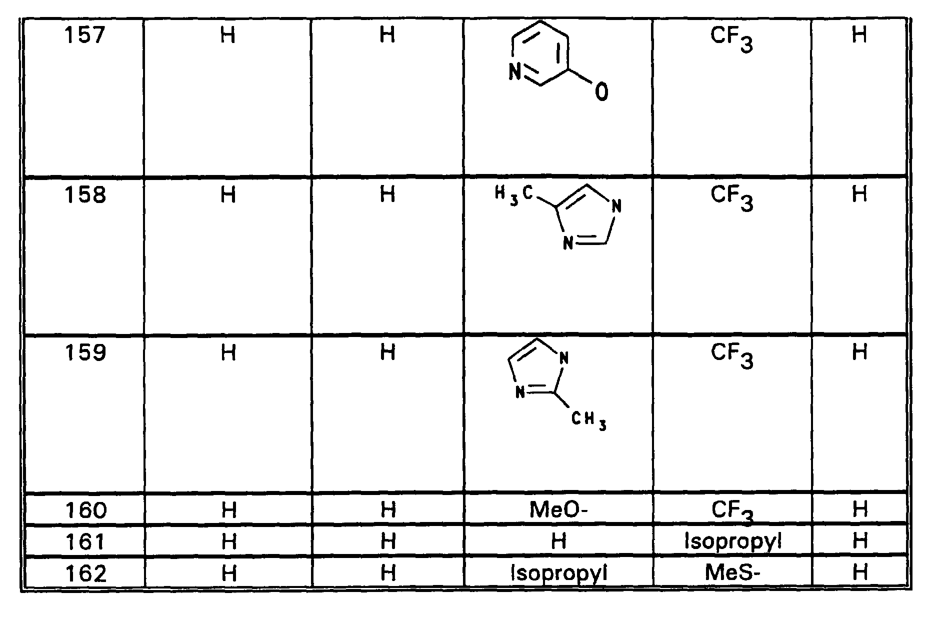

## Patentansprüche

1. Di-benzoylguanidine der Formel I a 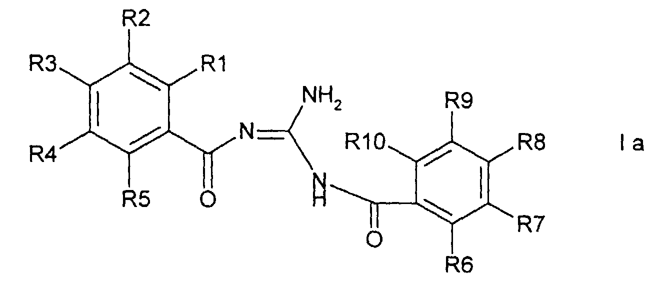 worin bedeuten:
R(1), R(5), R(6) und R(10)
unabhängig voneinander Wasserstoff, F, Cl, (C₁-C₃)-Alkyl, -OR(11), CᵣF_{2r + 1} oder -NR(11)R(12);
R(11) und R(12)
unabhängig voneinander Wasserstoff oder (C₁-C₃)-Alkyl;
r 1 bis 4;
R(2), R(4), R(7) und R(9)
unabhängig voneinander Wasserstoff, F, Cl, Br, I, -C≡N, Xₒ-(CH₂)ₚ-(CF₂)_{q}-CF₃, R(13)-SOₘ, R(14)R(15)N-CO-, R(16)-CO- oder R(17)R(18)N-SO₂-;
X Sauerstoff, S oder NR(19);
R(19) Wasserstoff oder (C₁-C₃)-Alkyl;
m Null, 1 oder 2;
o Null oder 1;
p Null, 1 oder 2;
q Null, 1, 2, 3, 4, 5 oder 6;
R(13), R(14), R(16) und R(17)
unabhängig voneinander (C₁-C₈)-Alkyl, (C₃-C₆)-Alkenyl, -CₙH₂ₙ-R(20) oder CF₃;
n Null, 1, 2, 3 oder 4;
R(20) (C₃-C₇)-Cycloalkyl oder Phenyl,
welches nicht substituiert ist oder substituiert mit 1 - 3 Substituenten aus der Gruppe F, Cl, CF₃, Methyl, Methoxy und NR(21)R(22);
R(21) und R(22)
H oder C₁-C₄-Alkyl,
oder
R(14), R(16) und R(17)
auch H;
R(15) und R(18)
unabhängig voneinander Wasserstoff oder (C₁-C₄)-Alkyl;
oder
R(14) und R(15) sowie R(17) und R(18)
gemeinsam 4 oder 5 Methylengruppen,
von denen eine CH₂-Gruppe durch Sauerstoff, S, NH, N-CH₃ oder N-Benzyl ersetzt sein kann;
oder
R(2), R(4), R(7) und R(9)
unabhängig voneinander (C₁-C₈)-Alkyl oder -CₐₗH₂ₐₗR(84);
al Null, 1 oder 2;
R(84) (C₃-C₈)-Cycloalkyl oder Phenyl,
welches nicht substituiert oder substituiert ist mit 1 - 3 Substituenten aus der Gruppe F, Cl, CF₃, Methyl, Methoxy und NR(85)R(86);
R(85) und R(86)
Wasserstoff oder CH₃;
oder
R(2), R(4), R(7) und R(9)
unabhängig voneinander Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl,
die über C oder N verknüpft sind und die unsubstituiert oder substituiert sind mit 1 - 3 Substituenten aus der Gruppe F, Cl, CF₃, CH₃, Methoxy, Hydroxy, Amino, Methylamino und Dimethylamino;
oder
R(2), R(4), R(7) und R(9)
unabhängig voneinander -C≡CR(93),
R(93) Phenyl,
das unsubstituiert oder substituiert ist mit 1 - 3 Substituenten aus der Gruppe F, Cl, CF₃, Methyl, Methoxy und NR(94)R(95);
R(94) und R(95)
Wasserstoff oder CH₃;
R(3) und R(8)
unabhängig voneinander wie R(2) definiert,
oder
R(3) und R(8)
unabhängig voneinander -Y-(p-C₆H₄)-(CO)ₕ-(CHOH)ᵢ-(CH₂)ⱼ-(CHOH)ₖ-R(23), -Y-(m-C₆H₄)-(CO)_{ad}-(CHOH)ₐₑ-(CH₂)_{af}-(CHOH)_{ag}-R(24) oder -Y-(o-C₆H₄)-(CO)ₐₕ-(CHOH)ₐₒ-(CH₂)ₐₚ-(CHOH)ₐₖ-R(25),
Y Sauerstoff, -S- oder -NR(83)-;
h, ad, ah
unabhängig voneinander Null oder 1;
i, j, k, ae, af, ag, ao, ap und ak
unabhängig voneinander Null, 1, 2, 3 oder 4;
wobei jedoch jeweils h, i und k nicht gleichzeitig Null,
ad, ae und ag nicht gleichzeitig Null sowie
ah, ao und ak nicht gleichzeitig Null sind,
R(23), R(24) R(25) und R(83)
unabhängig voneinander Wasserstoff oder (C₁-C₃)-Alkyl;
oder
R(3) und R(8)
unabhängig voneinander Wasserstoff, F, Cl, Br, I, CN (C₁-C₈)-Alkyl, (C₁-C₈)-Perfluoralkyl, (C₃-C₈)-Alkenyl, -C_{g}H_{2g}R(26),
g Null, 1, 2, 3 oder 4;
R(26) (C₃-C₈)-Cycloalkyl, Phenyl, Biphenylyl oder Naphthyl,
wobei die Aromaten nicht substituiert oder substituiert sind mit 1 - 3 Substituenten aus der Gruppe F, Cl, CF₃, Methyl, Methoxy und NR(27)R(28);
R(27) und R(28)
Wasserstoff, (C₁-C₄)-Alkyl oder (C₁-C₄)-Perfluoralkyl;
oder
R(3) und R(8)
unabhängig voneinander SR(29), -OR(30), -NR(31)R(32) oder -CR(33)R(34)R(35);
R(29), R(30), R(31) und R(33)
unabhängig voneinander -CₐH₂ₐ-[Furanyl, Thienyl, Pyrrolyl, Imidazolyl, Pyrazolyl, Traizolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl],
die unsubstituiert oder substituiert sind mit 1 - 3 Substituenten aus der Gruppe F, Cl, CF₃, CH₃, Methoxy, Hydroxy, Amino, Methylamino und Dimethylamino,
a Null, 1 oder 2;
R(32), R(34) und R(35)
unabhängig voneinander wie R(29) definiert oder Wasserstoff, (C₁-C₄)-Alkyl oder (C₁-C₄)-Perfluoralkyl;
oder
R(3) und R(8)
unabhängig voneinander -W-(p-C₆H₄)-R(96), -W-(m-C₆H₄)-R(97) oder -W-(o-C₆H₄)-R(98);
R(96), R(97) und R(98)
unabhängig voneinander Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl,
die über C oder N verknüpft sind und die unsubstituiert oder substituiert sind mit 1 bis 3 Substituenten aus der Gruppe F, Cl, CF₃, CH₃, Methoxy, Hydroxy, Amino, Methylamino, Dimethylamino oder Benzyl;
W Sauerstoff, S oder NR(99)-;
R(99) Wasserstoff oder (C₁-C₄)-Alkyl;
oder
R(3) und R(8)
unabhängig voneinander R(72)-SOₘ oder R(73)R(74)N-SO₂-;
m 1 oder 2;
R(72) (C₁-C₈)-Alkyl, (C₁-C₈)-Perfluoralkyl, (C₃-C₈)-Alkenyl oder -CₛH₂ₛ-R(75),
s Null, 1, 2, 3 oder 4;
R(75) (C₃-C₈)-Cycloalkyl, Phenyl, Biphenylyl oder Naphthyl,
wobei die Aromaten nicht substituiert oder substituiert sind mit 1 - 3 Substituenten aus der Gruppe F, Cl, CF₃, Methyl, Methoxy und NR(76)R(77);
R(76) und R(77)
Wasserstoff, (C₁-C₄)-Alkyl oder (C₁-C₄)-Perfluoralkyl;
R(73) Wasserstoff, (C₁-C₈)-Alkyl, (C₁-C₈)-Perfluoralkyl, (C₃-C₈)-Alkenyl oder -C_{w}H_{2w}-R(78),
w Null, 1, 2, 3 oder 4;
R(78) (C₃-C₈)-Cycloalkyl, Phenyl, Biphenylyl oder Naphthyl,
wobei die Aromaten nicht substituiert oder substituiert sind mit 1 - 3 Substituenten aus der Gruppe F, Cl, CF₃, Methyl, Methoxy und NR(79)R(80);
R(79) und R(80)
Wasserstoff, (C₁-C₄)-Alkyl oder (C₁-C₄)-Perfluoralkyl;
R(74) Wasserstoff, (C₁-C₄)-Alkyl oder (C₁-C₄)-Perfluoralkyl,
oder
R(73) und R(74)
gemeinsam 4 oder 5 Methylengruppen, von denen eine CH₂-Gruppe durch Sauerstoff, S, NH, N-CH₃ oder N-Benzyl ersetzt sein kann;
oder
R(3) und R(8)
unabhängig voneinander R(39)X-,
X Sauerstoff, S, NR(40), (D = O)A-, NR(41)C = MN⁽*⁾R(42)-;
M Sauerstoff oder S;
A Sauerstoff oder NR(43);
D C oder SO;
R(39) (C₁-C₈)-Alkyl, (C₃-C₈)-Alkenyl, (CH₂)_{b}C_{d}F_{2d + 1} oder -CₓH₂ₓ-R(44);
b Null oder 1;
d 1, 2, 3, 4, 5, 6 oder 7;
x Null, 1, 2, 3, oder 4;
R(44) (C₃-C₈)-Cycloalkyl, Phenyl, Biphenylyl oder Naphthyl,
wobei die Aromaten nicht substituiert oder substituiert sind mit 1 - 3 Substituenten aus der Gruppe F, Cl, CF₃, Methyl, Methoxy und NR(45)R(46);
R(45) und R(46)
Wasserstoff, (C₁-C₄)-Alkyl oder (C₁-C₄)-Perfluoralkyl;
R(40), R(41) und R(43)
unabhängig voneinander Wasserstoff, (C₁-C₄)-Alkyl oder (C₁-C₄)-Perfluoralkyl;
R(42) definiert wie R(39);
oder
R(39) und R(40) beziehungsweise R(39) und R(41)
gemeinsam 4 oder 5 Methylengruppen,
von denen eine CH₂-Gruppe durch Sauerstoff, S, NH, N-CH₃ oder N-Benzyl ersetzt sein kann;
wobei A und N⁽*⁾ an den Phenylkern des Benzoylguanidin-Grundkörpers gebunden sind;
oder
R(3) und R(8)
unabhängig voneinander -SR(47), -OR(48), -NHR(49), -NR(50)R(51), -CHR(52)R(53), -CR(54)R(55)OH, -C≡C-R(56), -CR(58) = CHR(57) oder -[CR(59)R(60)]ᵤ-CO-[CR(61)R(62)]ᵥ-R(63);
R(47), R(48), R(49), R(50) und R(52)
gleich oder verschieden -(CH₂)_{y}-(CHOH)_{z}-(CH₂)ₐₐ-(CHOH)ₜ-R(64) oder -(CH₂)_{ab}-O-(CH₂-CH₂O)_{ac}-R(65);
R(64) und R(65)
Wasserstoff oder Methyl;
u 1, 2, 3 oder 4;
v Null, 1, 2, 3 oder 4;
y, z und aa
gleich oder verschieden Null, 1, 2, 3 oder 4;
t = 1, 2, 3 oder 4;
R(51), R(53), R(54) und R(55)
gleich oder verschieden Wasserstoff oder (C₁-C₆)-Alkyl;
oder
R(52) und R(53) beziehungsweise R(54) und R(55)
zusammen mit dem sie tragenden Kohlenstoff-Atom ein (C₃-C₈)-Cycloalkyl;
R(63) Wasserstoff, (C₁-C₆)-Alkyl, (C₃-C₈)-Cycloalkyl oder -CₑH₂ₑ-R(81);
e Null, 1, 2, 3 oder 4;
R(56), R(57) und R(81)
unabhängig Phenyl,
das unsubstituiert oder substituiert ist mit 1 - 3 Substituenten aus der Gruppe F, Cl, CF₃, Methyl, Methoxy oder NR(82)R(66) mit R(82) und R(66) gleich H oder (C₁-C₄)-Alkyl;
oder
R(56), R(57) und R(81)
unabhängig voneinander Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl],
die unsubstituiert oder wie Phenyl substituiert sind;
R(58), R(59), R(60), R(61) und R(62).
Wasserstoff oder Methyl;
oder
R(3) und R(8)
unabhängig voneinander R(67)-NH-SO₂-;
R(67) R(68)R(69)N-(C = Y')-;
Y' Sauerstoff, S oder N-R(70);
R(68) und R(69)
gleich oder verschieden Wasserstoff, (C₁-C₈)-Alkyl, (C₃-C₆)-Alkenyl oder -C_{f}H_{2f}-R(71);
f Null, 1, 2, 3 oder 4;
R(71) (C₅-C₇)-Cycloalkyl oder Phenyl,
welches unsubstituiert ist oder substituiert mit 1 - 3 Substituenten aus der Gruppe F, Cl, CF₃, Methoxy und (C₁-C₄)-Alkyl;
oder
R(68) und R(69)
gemeinsam 4 oder 5 Methylengruppen,
von denen eine CH₂-Gruppe durch Sauerstoff, S, NH, N-CH₃ oder N-Benzyl ersetzt sein kann;
wobei
R(70) wie R(68) oder Amidin;
sowie deren pharmazeutisch verträgliche Salze,
wobei jedoch Verbindungen ausgenommen sind, in denen die Reste R(1) bis R(10) folgendermaßen kombiniert sind:
| R(1) | R(2) | R(3) | R(4) | R(5) | R(6) | R(7) | R(8) | R(9) | R(10) |
|---|---|---|---|---|---|---|---|---|---|
| H | Cl | Cl | H | H | H | H | Cl | Cl | H |
| H | H | NH₂ | H | H | H | H | NH₂ | H | H |
| H | H | H | H | H | H | H | H | H | H |
| Cl | H | H | H | H | H | H | H | H | Cl |
| H | H | Cl | H | H | H | H | Cl | H | H |
| H | H | CH₃ | H | H | H | H | CH₃ | H | H |
| H | H | NH₂ | H | H | H | H | H | H | H |
| H | H | Cl | H | H | H | H | H | H | H |
| H | H | CH₃ | H | H | H | H | H | H | H |

2. Verbindung der Formel I a nach Anspruch 1, in der bedeuten:
R(5) und R(6)
Wasserstoff;
die übrigen Reste wie in Anspruch 1 definiert sind
und
R(1) und R(10) gleich sind,
R(2) und R(9) gleich sind,
R(3) und R(8) gleich sind,
R(4) und R(7) gleich sind.

3. Verbindungen der Formel I a nach Anspruch 1, in denen bedeuten:
R(1) und R(10)
gleich sind und Wasserstoff, F, Cl, CH₃, OH, NH₂ oder CF₃;
R(2) und R(9)
gleich sind und Wasserstoff, F, Cl, Br, -C≡N, -C_{q}F_{2q}CF₃, R(13)-SO₂, R(14)R(15)N-CO-, R(16)-CO- oder R(17)R(18)N-SO₂-;
q Null, 1, 2, 3, 4 oder 5;
R(13), R(14), R(16) und R(17)
unabhängig voneinander (C₁-C₈)-Alkyl, (C₃-C₄)-Alkenyl, -CₙH₂ₙ-R(20) oder CF₃;
n Null oder 1;
R(20) (C₃-C₆)-Cycloalkyl oder Phenyl,
welches nicht substituiert ist oder substituiert mit 1 - 2 Substituenten aus der Gruppe F, Cl, CF₃, Methyl, Methoxy und NR(21)R(22);
R(21) und R(22)
Wasserstoff oder Methyl;
oder R(14), R(16) und R(17)
Wasserstoff;
R(15) und R(18)
unabhängig voneinander Wasserstoff oder Methyl;
R(3) und R(8)
gleich sind und -Y-(p-C₆H₄)-(CO)ₕ-(CHOH)ᵢ-(CH₂)ⱼ-(CHOH)ₖ-R(23), -Y-(m-C₆H₄)-(CO)_{ad}-(CHOH)ₐₑ-(CH₂)_{af}-(CHOH)_{ag}-R(24) oder -Y-(o-C₆H₄)-(CO)ₐₕ-(CHOH)ₐₒ-(CH₂)ₐₚ-(CHOH)ₐₖ-R(25);
Y Sauerstoff, S oder -NR(83);
R(23), R(24), R(25) und R(83)
unabhängig voneinander Wasserstoff oder Methyl;
h, ad und ah
unabhängig voneinander Null oder 1;
i, k, ae, ag, ao und ak
unabhängig voneinander Null, 1, 2 oder 3;
j, af und ap
unabhängig voneinander Null oder 1;
wobei jedoch jeweils h, i und k nicht gleichzeitig Null sind,
ad, ae und ag nicht gleichzeitig Null sind und
ah, ao und ak nicht gleichzeitig Null sind,
oder
R(3) und R(8)
gleich sind und Wasserstoff, F, Cl, Br, CN, (C₁-C₈)-Alkyl, CF₃, (C₃-C₈)-Alkenyl oder -C_{g}H_{2g}R(26);
g Null, 1 oder 2;
R(26) (C₃-C₆)-Cycloalkyl oder Phenyl,
welches nicht substituiert oder substituiert ist mit 1 - 2 Substituenten aus der Gruppe F, Cl, CF₃, Methyl, Methoxy und NR(27)R(28);
R(27) und R(28)
H oder CH₃;
oder
R(3) und R(8)
gleich sind und Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl;
die über C oder N verknüpft sind und die unsubstituiert oder substituiert sind mit 1 - 2 Substituenten aus der Gruppe F, Cl, CF₃, CH₃, Methoxy, Hydroxy, Amino, Methylamino und Dimethylamino;
oder
R(3) und R(8)
gleich sind und -W-(p-C₆H₄)-R(96), -W-(m-C₆H₄)-R(97) oder -W-(o-C₆H₄)-R(98);
R(96), R(97 und R(98)
unabhängig voneinander Pyrrolyl, Imidazolyl, Pyrazolyl oder Pyridyl,
das jeweils unsubstituiert oder substituiert ist mit 1 bis 2 Resten aus der Reihe F, Cl, CF₃, CH₃, Methoxy, Dimethylamino und Benzyl;
W Sauerstoff, -S- oder NR(99)-;
R(99) Wasserstoff oder Methyl;
oder
R(3) und R(8)
gleich sind und -SR(29), -OR(30), -NR(31)R(32) oder -CR(33)R(34)R(35); R(29), R(30), R(31) und R(33)
unabhängig voneinander -CₐH₂ₐ-[Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl],
die unsubstituiert oder substituiert sind mit 1 - 2 Substituenten aus der Gruppe F, Cl, CF₃, CH₃, Methoxy, Hydroxy, Amino, Methylamino und Dimethylamino;
a Null oder 1;
R(32), R(34) und R(35)
unabhängig voneinander Wasserstoff oder CH₃;
oder
R(3) und R(8)
gleich sind und R(72)-SO₂ oder R(73)R(74)N-SO₂-;
R(72) (C₁-C₄)-Alkyl, CF₃, (C₃-C₄)-Alkenyl oder -CₛH₂ₛ-R(75);
s Null oder 1;
R(75) (C₃-C₆)-Cycloalkyl oder Phenyl,
welches nicht substituiert oder substituiert ist mit 1 - 2 Substituenten aus der Gruppe F, Cl, CF₃, Methyl, Methoxy und NR(76)R(77);
R(76) und R(77)
Wasserstoff oder CH₃,
R(73) Wasserstoff, (C₁-C₄)-Alkyl, CF₃, (C₃-C₄)-Alkenyl oder -C_{w}H_{2w}-R(78);
w Null oder 1;
R(78) (C₃-C₆)-Cycloalkyl oder Phenyl,
welches nicht substituiert oder substituiert ist mit 1 - 2 Substituenten aus der Gruppe F, Cl, CF₃, Methyl, Methoxy und NR(79)R(80);
R(79) und R(80)
Wasserstoff oder CH₃;
R(74) Wasserstoff oder CH₃;
oder
R(73) und R(74)
gemeinsam 4 oder 5 Methylengruppen,
von denen eine CH₂-Gruppe durch Sauerstoff, S, NH, N-CH₃ oder N-Benzyl ersetzt sein kann;
oder
R(3) und R(8)
gleich sind und R(39)X-;
X Sauerstoff, S, NR(40), (C = O)A- oder NR(41)C = MN⁽*⁾R(42)-,
M Sauerstoff;
A Sauerstoff oder NR(43);
R(43) Wasserstoff, (C₁-C₄)-Alkyl oder (C₁-C₄)-Perfluoralkyl;
R(41) Wasserstoff, (C₁-C₄)-Alkyl,
R(42) definiert wie R(39);
R(39) (C₁-C₆)-Alkyl, (C₃-C₄)-Alkenyl, (CH₂)_{b}C_{d}F_{2d + 1} oder -CₓH₂ₓ-R(44);
b Null oder 1;
d 1 - 7;
x Null oder 1,
R(44) (C₃-C₆)-Cykloalkyl oder Phenyl,
welches nicht substituiert oder substituiert ist mit 1 - 2 Substituenten aus der Gruppe F, Cl, CF₃, Methyl, Methoxy und NR(45)R(46);
R(45) und R(46)
Wasserstoff oder CH₃;
oder
R(39) und R(40) beziehungsweise R(39) und R(41)
gemeinsam 4 oder 5 Methylengruppen,
von denen eine CH₂-Gruppe durch Sauerstoff, S, NH, N-CH₃ oder N-Benzyl ersetzt sein kann;
wobei A und N⁽*⁾ an den Phenylkern des Benzoylguanidin-Grundkörpers gebunden sind;
oder
R(3) und R(8)
gleich sind und -SR(47), -OR(48), -NHR(49), -NR(50)R(51), -CHR(52)R(53), -CR(54)R(55)OH, -C≡C-R(56), -CR(58) = CHR(57) oder -[CR(59)R(60)]ᵤ-CO-[CR(61)R(62)]ᵥ-R(63);
R(47), R(48), R(49), R(50) und R(52)
gleich oder verschieden -(CH₂)_{y}-(CHOH)_{z}-(CH₂)ₐₐ-(CHOH)ₜ-R(64) oder -(CH₂)_{ab}-O-(CH₂-CH₂O)_{ac}-R(65);
y, z, aa, ab, ac
gleich oder verschieden Null, 1 oder 2;
R(64) und R(65)
Wasserstoff oder Methyl;
u 1 oder 2;
v Null, 1 oder 2;
t 1, 2 oder 3;
R(51), R(53), R(54) und R(55)
gleich oder verschieden Wasserstoff oder Methyl;
oder
R(52) und R(53) beziehungsweise R(54) und R(55)
zusammen mit dem sie tragenden Kohlenstoff-Atom ein (C₃-C₆)-Cycloalkyl,
R(63) Wasserstoff, (C₁-C₄)-Alkyl, (C₃-C₆)-Cycloalkyl oder -CₑH₂ₑ-R(81);
e Null, 1 oder 2;
R(56), R(57), R(81)
unabhängig voneinander Phenyl,
das unsubstituiert oder substituiert ist mit 1 - 2 Substituenten aus der Gruppe F, Cl, CF₃, Methyl, Methoxy oder NR(65)R(66) mit R(65) und R(66) gleich Wasserstoff oder CH₃;
oder
R(56), R(57) und R(81)
unabhängig voneinander Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl oder Cinnolinyl,
die unsubstituiert oder wie Phenyl substituiert sind;
R(58), R(59), R(60), R(61) und R(62)
Wasserstoff, Methyl;
oder
R(3) und R(8)
R(67)-NH-SO₂-;
R(67) R(68)R(69)N-(C = Y')-;
Y' Sauerstoff, S oder N-R(70);
R(68) und R(69)
gleich oder verschieden Wasserstoff, (C₁-C₄)-Alkyl, (C₃-C₄)-Alkenyl oder -C_{f}H_{2f}-R(71);
f Null oder 1;
R(71) (C₅-C₇)-Cycloalkyl oder Phenyl,
welches unsubstituiert oder substituiert ist mit 1 - 2 Substituenten aus der Gruppe F, Cl, CF₃, Methoxy und Methyl;
oder
R(68) und R(69)
gemeinsam 4 oder 5 Methylengruppen,
von denen eine CH₂-Gruppe durch Sauerstoff, S, NH, N-CH₃ oder N-Benzyl ersetzt sein kann,
R(70) wie R(68) definiert ist;
R(4) und R(7)
gleich sind und (C₁-C₈)-Alkyl, -CₐₗH₂ₐₗR(84) oder CF₃;
al Null oder 1;
R(84) (C₃-C₆)-Cycloalkyl oder Phenyl,
welches nicht substituiert oder substituiert ist mit 1 - 2 Substituenten aus der Gruppe F, Cl, CF₃, Methyl, Methoxy und NR(85)R(86);
R(85) und R(86)
Wasserstoff oder CH₃;
oder
R(4) und R(7)
gleich sind und Chinolyl, Isochinolyl, Pyrrolyl, Pyridyl oder Imidazolyl,
die über C oder N verknüpft sind und die unsubstituiert oder substituiert sind mit 1 - 2 Substituenten aus der Gruppe F, Cl, CF₃, CH₃, Methoxy, Hydroxy, Amino, Methylamino und Dimethylamino;
oder
R(4) und R(7)
gleich sind und R(87)-SOₐₘ oder R(88)R(89)N-SO₂-;
am 2;
R(87) (C₁-C₄)-Alkyl oder CF₃;
R(88) Wasserstoff, (C₁-C₄)-Alkyl, CF₃ oder -CₐₙH₂ₐₙ-R(90),
an Null oder 1;
R(90) Phenyl,
welches nicht substituiert oder substituiert ist mit 1 - 2 Substituenten aus der Gruppe F, Cl, CF₃, Methyl, Methoxy und NR(90)R(91);
R(90) und R(91)
Wasserstoff oder CH₃;
R(89) Wasserstoff oder CH₃;
oder
R(4) und R(7)
gleich sind und -C≡CR(93);
R(93) Phenyl,
das unsubstituiert oder substituiert ist mit 1 - 2 Substituenten aus der Gruppe F, Cl, CF₃, Methyl, Methoxy und NR(94)R(95);
R(94) und R(95)
Wasserstoff oder CH₃;
R(5) und R(6)
gleich sind und Wasserstoff;
sowie deren pharmazeutisch verträgliche Salze.

4. Verfahren zur Herstellung einer Verbindung I a nach Anspruch 1, dadurch gekennzeichnet, daß man
a) je ein Äquivalent der Verbindungen der Formel II aa und II bb mit einem Äquivalent Guanidin umsetzt, 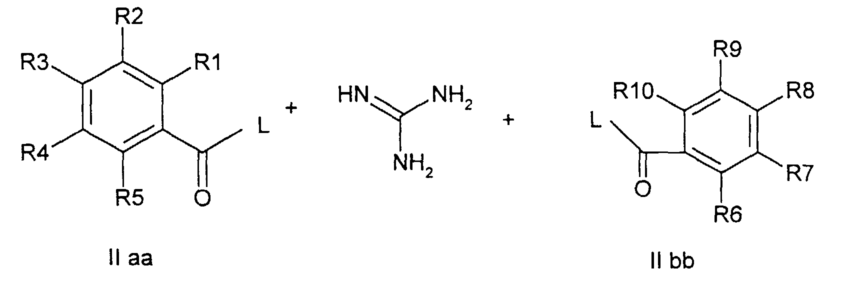 worin R(1) bis R(10) die angegebene Bedeutung besitzen und L für eine leicht nucleophil substituierbare leaving group steht, oder
b) eine Verbindung der Formel II aa 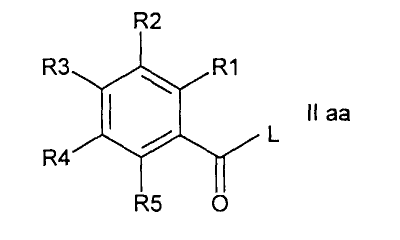 mit einer Verbindung der Formel III aa 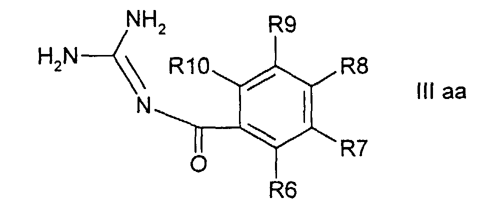 unter Beteiligung einer Base umsetzt, worin R(1) bis R(10) die angegebenen Bedeutungen besitzen und L für eine leicht nucleophil substituierbare leaving group steht,
und daß man gegebenenfalls in ein pharmazeutisch verträgliches Salz überführt.

5. Verwendung einer Verbindung I a nach Anspruch 1 zur Herstellung eines Medikaments zur Behandlung von Arrhythmien.

6. Verwendung einer Verbindung I a nach Anspruch 1 zur Herstellung eines Medikaments zur Behandlung oder Prophylaxe des Herzinfarkts.

7. Verwendung einer Verbindung I a nach Anspruch 1 zur Herstellung eines Medikaments zur Behandlung oder Prophylaxe der Angina Pectoris.

8. Verwendung einer Verbindung I a nach Anspruch 1 zur Herstellung eines Medikaments zur Behandlung oder Prophylaxe von ischämischen Zuständen des Herzens.

9. Verwendung einer Verbindung I a nach Anspruch 1 zur Herstellung eines Medikaments zur Behandlung oder Prophylaxe von ischämischen Zuständen des peripheren und zentralen Nervensystems und des Schlaganfalls.

10. Verwendung einer Verbindung I a nach Anspruch 1 zur Herstellung eines Medikaments zur Behandlung oder Prophylaxe von ischämischen Zuständen peripherer Organe und Gliedmaßen.

11. Verwendung einer Verbindung I a nach Anspruch 1 zur Herstellung eines Medikaments zur Behandlung von Schockzuständen.

12. Verwendung einer Verbindung I a nach Anspruch 1 zur Herstellung eines Medikaments zum Einsatz bei chirurgischen Operationen und Organtransplantationen.

13. Verwendung einer Verbindung I a nach Anspruch 1 zur Herstellung eines Medikaments zur Konservierung und Lagerung von Transplantaten für chirurgische Maßnahmen.

14. Verwendung einer Verbindung I a nach Anspruch 1 zur Herstellung eines Medikaments zur Behandlung von Krankheiten, bei denen die Zellproliferation eine primäre oder sekundäre Ursache darstellt.

15. Heilmittel, enthaltend eine wirksame Menge einer Verbindung I a nach Anspruch 1.

## Claims

1. A dibenzoylguanidine of the formula I a 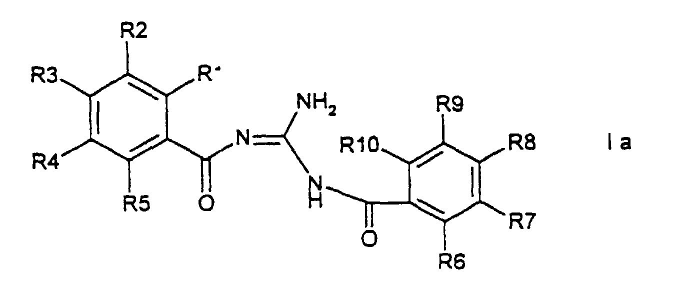 in which:
R(1), R(5), R(6) and R(10)
independently of one another are hydrogen, F, Cl, (C₁-C₃)-alkyl, -OR(11), CᵣF₂ᵣ₊₁ or -NR(11)R(12);
R(11) and R(12)
independently of one another are hydrogen or (C₁-C₃)-alkyl;
r is 1 to 4;
R(2), (R(4), R(7) and R(9)
independently of one another are hydrogen, F, Cl, Br, I, -C≡N, Xₒ-CH₂)ₚ-(CF₂)_{q}-CF₃, R(13)-SOₘ, R(14)R(15)N-CO-, R(16)-CO- or R(17)R(18)N-SO₂-;
X is oxygen, S or NR(19);
R(19) is hydrogen or (C₁-C₃)-alkyl;
m is 0, 1 or 2;
o is 0 or 1;
p is 0, 1 or 2;
q is 0, 1, 2, 3, 4, 5 or 6;
R(13), R(14), R(16) and R(17)
independently of one another are (C₁-C₈)-alkyl, (C₃-C₆)-alkenyl, -CₙH₂ₙ-R(20) or CF₃; n is zero, 1, 2, 3 or 4;
R(20) is (C₃-C₇)-cycloalkyl or phenyl,
which is not substituted or is substituted by 1-3 substituents from the group consisting of F, Cl, CF₃, methyl, methoxy and NR(21)R(22);
R(21) and R(22)
are H or C₁-C₄-alkyl,
or
R(14), R(16) and R(17)
are also H;
R(15) and R(18)
independently of one another are hydrogen or (C₁-C₄)-alkyl;
or
R(14) and R(15) and also R(17) and R(18)
together are 4 or 5 methylene groups,
of which one CH₂ group can be replaced by oxygen, S, NH, N-CH₃ or N-benzyl;
or
R(2), R(4), R(7) and R(9)
independently of one another are (C₁-C₈)-alkyl or -CₐₗH₂ₐₗR(84);
al is zero, 1 or 2;
R(84) is (C₃-C₈)-cycloalkyl or phenyl,
which is not substituted or is substituted by 1-3 substituents from the group consisting of F, Cl, CF₃, methyl, methoxy and NR(85)R(86);
(R(85) and R(86)
are hydrogen or CH₃;
or
R(2), R(4), R(7) and R(9)
independently of one another are furanyl, thienyl, pyrrolyl, imidazolyl, pyrazolyl, triazolyl, tetrazolyl, oxazolyl, isoxazolyl, thiazolyl, isothiazolyl, pyridyl, pyrazinyl, pyrimidinyl, pyridazinyl, indolyl, indazolyl, quinolyl, isoquinolyl, phthalazinyl, quinoxalinyl, quinazolinyl or cinnolinyl,
which are linked via C or N and which are unsubstituted or substituted by 1-3 substituents from the group consisting of F, Cl, CF₃, CH₃, methoxy, hydroxyl, amino, methylamino and dimethylamino;
or
R(2), R(4), R(7) and R(9)
independently of one another are -C≡CR(93),
R(93) is phenyl,
which is unsubstituted or substituted by 1-3 substituents from the group consisting of F, Cl, CF₃, methyl, methoxy and NR(94)R(95);
R(94) and R(95)
are hydrogen or CH₃;
R(3) and R(8)
independently of one another are defined as R(2),
or
R(3) and R(8)
independently of one another are -Y-(p-C₆H₄)-(CO)ₕ-(CHOH)ᵢ-(CH₂)ⱼ-(CHOH)ₖ-R(23), -Y-(m-C₆H₄)-(CO)_{ad}-(CHOH)ₐₑ-(CH₂)_{af}-(CHOH)_{ag}-R(24) or -Y-(o-C₆H₄)-(CO)ₐₕ-(CHOH)ₐₒ-(CH₂)ₐₚ-(CHOH)ₐₖ-R(25),
Y is oxygen, -S- or -NR(83)-;
h, ad, ah
independently of one another are zero or 1;
i, j, k, ae, af, ag, ao, ap and ak
independently of one another are zero, 1, 2, 3 or 4;
but where h, i and k in each case are not simultaneously zero,
ad, ae and ag are not simultaneously zero and
ah, ao and ak are not simultaneously zero,
R(23), R(24), R(25) and R(83)
independently of one another are hydrogen or (C₁-C₃)-alkyl;
or
R(3) and R(8)
independently of one another are hydrogen, F, Cl, Br, I, CN, (C₁-C₈)-alkyl, (C₁-C₈)-perfluoroalkyl, (C₃-C₈)-alkenyl, -C_{g}H_{2g}R(26),
g is zero, 1, 2, 3 or 4;
R(26) is (C₃-C₈)-cycloalkyl, phenyl, biphenylyl or naphthyl,
where the aromatics are not substituted or are substituted by 1-3 substituents from the group consisting of F, Cl, CF₃, methyl, methoxy and NR(27)R(28);
R(27) and R(28)
are hydrogen, (C₁-C₄)-alkyl or (C₁-C₄)-perfluoroalkyl;
or
R(3) and R(8)
independently of one another are SR(29), -OR(30), -NR(31)R(32) or -CR(33)R(34)R(35);
R(29), R(30), R(31) and R(33)
independently of one another are -CₐH₂ₐ-[furanyl, thienyl, pyrrolyl, imidazolyl, pyrazolyl, triazolyl, tetrazolyl, oxazolyl, isoxazolyl, thiazolyl, isothiazolyl, pyridyl, pyrazinyl, pyrimidinyl, pyridazinyl, indolyl, indazolyl, quinolyl, isoquinolyl, phthalazinyl, quinoxalinyl, quinazolinyl or cinnolinyl],
which are unsubstituted or substituted by 1-3 substituents from the group consisting of F, Cl, CF₃, CH₃, methoxy, hydroxyl, amino, methylamino and dimethylamino,
a is zero, 1 or 2;
R(32), R(34) and R(35)
independently of one another are defined as R(29) or are hydrogen, (C₁-C₄)-alkyl or (C₁-C₄)-perfluoroalkyl;
or
R(3) and R(8)
independently of one another are -W-(p-C₆H₄)-R(96), -W-(m-C₆H₄)-R(97) or -W-(o-C₆H₄)-R(98);
R(96), R(97) and R(98)
independently of one another are furanyl, thienyl, pyrrolyl, imidazolyl, pyrazolyl, triazolyl, tetrazolyl, oxazolyl, isoxazolyl, thiazolyl, isothiazolyl, pyridyl, pyrazinyl, pyrimidinyl, pyridazinyl, indolyl, indazolyl, quinolyl, isoquinolyl, phthalazinyl, quinoxalinyl, quinazolinyl or cinnolinyl,
which are linked via C or N and which are unsubstituted or substituted by 1 to 3 substituents from the group consisting of F, Cl, CF₃, CH₃, methoxy, hydroxyl, amino, methylamino, dimethylamino or benzyl;
W is oxygen, S or NR(99)-;
R(99) is hydrogen or (C₁-C₄)-alkyl;
or
R(3) and R(8)
independently of one another are R(72)-SOₘ or R(73)R(74)N-SO₂-;
m is 1 or 2;
R(72) is (C₁-C₈)-alkyl, (C₁-C₈)-perfluoroalkyl, (C₃-C₈)-alkenyl or -CₛH₂ₛ-R(75),
s is zero, 1, 2, 3 or 4;
R(75) is (C₃-C₈)-cycloalkyl, phenyl, biphenylyl or
naphthyl, where the aromatics are not substituted or are substituted by 1-3 substituents from the group consisting of F, Cl, CF₃, methyl, methoxy and NR(76)R(77);
R(76) and R(77)
are hydrogen, (C₁-C₄)-alkyl or (C₁-C₄)-perfluoroalkyl;
R(73) is hydrogen, (C₁-C₈)-alkyl, (C₁-C₈)-perfluoroalkyl, (C₃-C₈)-alkenyl or -C_{w}H_{2w}-R(78),
w is zero, 1, 2, 3 or 4;
R(78) is (C₃-C₈)-cycloalkyl, phenyl,
biphenylyl or naphthyl, where the aromatics are not substituted or are substituted by 1-3 substituents from the group consisting of F, Cl, CF₃, methyl, methoxy and NR(79)R(80);
R(79)R(80)
are hydrogen, (C₁-C₄)-alkyl or (C₁-C₄)-perfluoroalkyl;
R(74) is hydrogen, (C₁-C₄)-alkyl or (C₁-C₄)-perfluoroalkyl
or
R(73) and R(74)
together are 4 or 5 methylene groups, of which one CH₂ group can be replaced by oxygen, S, NH, N-CH₃ or N-benzyl;
or
R(3) and R(8)
independently of one another are R(39)X-,
X is oxygen, S, NR(40), (D=O)A-, NR(41)C=MN⁽*⁾R(42)-;
M is oxygen or S;
A is oxygen or NR(43);
D is C or SO;
R(39) is (C₁-C₈)-alkyl, (C₃-C₈)-alkenyl, (CH₂)_{b}C_{d}F_{2d+1} or -CₓH₂ₓ-R(44);
b is zero or 1;
d is 1, 2, 3, 4, 5, 6 or 7;
x is zero, 1, 2, 3 or 4;
R(44) is (C₃-C₈)-cycloalkyl, phenyl,
biphenylyl or naphthyl, where the aromatics are not substituted or are substituted by 1-3 substituents from the group consisting of F, Cl, CF₃, methyl, methoxy and NR(45)R(46);
R(45) and R(46)
are hydrogen, (C₁-C₄)-alkyl or (C₁-C₄)-perfluoroalkyl;
R(40), R(41) and R(43)
independently of one another are hydrogen, (C₁-C₄)-alkyl or (C₁-C₄)-perfluoroalkyl;
R(42) is defined as R(39);
or
R(39) and R(40) or R(39) and R(41)
together are 4 or 5 methylene groups,
of which one CH₂ group can be replaced by oxygen, S, NH, N-CH₃ or N-benzyl;
where A and N⁽*⁾ are bonded to the phenyl nucleus of the benzoylguanidine parent structure;
or
R(3) and R(8)
independently of one another are -SR(47), -OR(48), -NHR(49), -NR(50)R(51), -CHR(52)R(53), -CR(54)R(55)OH, -C≡C-R(56), -CR(58)=CHR(57) or -[CR(59)R(60)] ᵤ-CO-[CR(61)R(62)]ᵥ-R(63);
R(47), R(48), R(49), R(50) and R(52)
are identical or different and are -(CH₂)_{y}-(CHOH)_{z}-(CH₂)ₐₐ-(CHOH)ₜ-R(64) or -(CH₂)_{ab}-O-(CH₂-CH₂O)_{ac}-R(65);
R(64) and R(65)
are hydrogen or methyl;
u is 1, 2, 3 or 4;
v is zero, 1, 2, 3 or 4;
y, z and aa
are identical or different and are zero, 1, 2, 3 or 4;
t = 1, 2, 3 or 4;
R(51), R(53), R(54) and R(55)
are identical or different and are hydrogen or (C₁-C₆)-alkyl;
or
R(52) and R(53) or R(54) and R(55)
together with the carbon atom carrying them are a (C₃-C₈)-cycloalkyl;
R(63) is hydrogen, (C₁-C₆)-alkyl, (C₃-C₈)-cycloalkyl or -CₑH₂ₑ-R(81);
e is zero, 1, 2, 3 or 4;
R(56), R(57) and R(81)
independently of one another are phenyl,
which is unsubstituted or is substituted by 1-3 substituents from the group consisting of F, Cl, CF₃, methyl, methoxy or NR(82)R(66) where R(82) and R(66) are H or (C₁-C₄)-alkyl;
or
R(56), R(57) and R(81)
independently of one another are furanyl, thienyl, pyrrolyl, imidazolyl, pyrazolyl, triazolyl, tetrazolyl, oxazolyl, isoxazolyl, thiazolyl, isothiazolyl, pyridyl, pyrazinyl, pyrimidinyl, pyridazinyl, indolyl, indazolyl, quinolyl, isoquinolyl, phthalazinyl, quinoxalinyl, quinazolinyl or cinnolinyl,
which are unsubstituted or are substituted as phenyl;
R(58), R(59), R(60), R(61) and R(62)
are hydrogen or methyl;
or
R(3) and R(8)
independently of one another are R(67)-NH-SO₂-;
R(67) is R(68)R(69)N-(C=Y')-;
Y' is oxygen, S or N-R(70);
R(68) and R(69)
are identical or different and are hydrogen, (C₁-C₈)-alkyl, (C₃-C₆)-alkenyl or -C_{f}H_{2f}-R(71);
f is zero, 1, 2, 3 or 4;
R(71) is (C₅-C₇)-cycloalkyl or phenyl,
which is unsubstituted or is substituted by 1-3 substituents from the group consisting of F, Cl, CF₃, methoxy and (C₁-C₄)-alkyl;
or
(R68) and R(69)
together are 4 or 5 methylene groups,
of which one CH₂ group can be replaced by oxygen, S, NH, N-CH₃ or N-benzyl;
where
R(70) is as R(68) or is amidine;
or its pharmaceutically tolerable salts,
but where compounds are excluded in which the radicals R(1) to R(10) are combined in the following manner:
| R(1) | R(2) | R(3) | R(4) | R(5) | R(6) | R(7) | R(8) | R(9) | R(10) |
|---|---|---|---|---|---|---|---|---|---|
| H | Cl | Cl | H | H | H | H | Cl | Cl | H |
| H | H | NH₂ | H | H | H | H | NH₂ | H | H |
| H | H | H | H | H | H | H | H | H | H |
| Cl | H | H | H | H | H | H | H | H | Cl |
| H | H | Cl | H | H | H | H | Cl | H | H |
| H | H | CH₃ | H | H | H | H | CH₃ | H | H |
| H | H | NH₂ | H | H | H | H | H | H | H |
| H | H | Cl | H | H | H | H | H | H | H |
| H | H | CH₃ | H | H | H | H | H | H | H |

2. A compound of the formula I a as claimed in claim 1, in which:
R(5) and R(6)
are hydrogen;
the other radicals are defined as in claim 1
and
R(1) and R(10) are identical,
R(2) and R(9) are identical,
R(3) and R(8) are identical,
R(4) and R(7) are identical.

3. A compound of the formula I as claimed in claim 1, in which:
R(1) and R(10)
are identical and are hydrogen, F, Cl, CH₃, OH, NH₂ or CF₃;
R(2) and R(9)
are identical and are hydrogen, F, Cl, Br, -C≡N, -C_{q}F_{2q}CF₃, R(13)-SO₂, R(14)R(15)N-CO-, R(16)-CO- or R(17)R(18)N-SO₂-;
q is zero, 1, 2, 3, 4 or 5;
R(13), R(14), R(16) and R(17)
independently of one another are (C₁-C₈)-alkyl, (C₃-C₄)-alkenyl, CₙH₂ₙ-R(20) or CF₃;
n is zero or 1;
R(20) is (C₃-C₆)-cycloalkyl or phenyl,
which is not substituted or is substituted by 1-2 substituents from the group consisting of F, Cl, CF₃, methyl, methoxy and NR(21)R(22);
R(21) and R(22)
are hydrogen or methyl;
or R(14), R(16) and R(17)
are hydrogen;
R(15) and R(18)
independently of one another are hydrogen or methyl;
R(3) and R(8)
are identical and are -Y-(p-C₆H₄)-(CO)ₕ-(CHOH)ᵢ-(CH₂)ⱼ-(CHOH)ₖ-R(23),
-Y-(m-C₆H₄)-(CO)_{ad}-(CHOH)ₐₑ-(CH₂)_{af}-(CHOH)_{ag}-R(24)
or -Y-(o-C₆H₄)-(CO)ₐₕ-(CHOH)ₐₒ-(CH₂)ₐₚ-(CHOH)ₐₖ-R(25);
Y is oxygen, S or -NR(83);
R(23), R(24), R(25) and R(83)
independently of one another are hydrogen or methyl;
h, ad and ah
independently of one another are zero or 1;
i, k, ae, ag, ao and ak
independently of one another are zero, 1, 2, or 3;
j, af and ap
independently of one another are zero or 1;
but where h, i and k in each case are not simultaneously zero and
ad, ae and ag are not simultaneously zero and
ah, ao and ak are not simultaneously zero,
or
R(3) and R(8)
are identical and are hydrogen, F, Cl, Br, CN, (C₁-C₈)-alkyl, CF₃, (C₃-C₈)-alkenyl or -C_{g}H_{2g}R(26);
g is zero, 1 or 2;
R(26) is (C₃-C₆)-cycloalkyl or phenyl,
which is not substituted or is substituted by 1-2 substituents from the group consisting of F, Cl, CF₃, methyl, methoxy and NR(27)R(28);
R(27) and R(28)
are H or CH₃;
or
R(3) and R(8)
are identical and are furanyl, thienyl, pyrrolyl, imidazolyl, pyrazolyl, triazolyl, tetrazolyl, oxazolyl, isoxazolyl, thiazolyl, isothiazolyl, pyridyl, pyrazinyl, pyrimidinyl, pyridazinyl, indolyl, indazolyl, quinolyl, isoquinolyl, phthalazinyl, quinoxalinyl, quinazolinyl or cinnolinyl;
which are linked via C or N and which are unsubstituted or substituted by 1-2 substituents from the group consisting of F, Cl, CF₃, CH₃, methoxy, hydroxyl, amino, methylamino and dimethylamino;
or
R(3) and R(8)
are identical and are -W-(p-C₆H₄)-R(96), -W-(m-C₆H₄)-R(97) or -W-(o-C₆H₄)-R(98);
R(96), R(97) and R(98)
independently of one another are pyrrolyl, imidazolyl, pyrazolyl or pyridyl,
which in each case is unsubstituted or substituted by 1 or 2 radicals from the group consisting of F, Cl, CF₃, CH₃, methoxy, dimethylamino and benzyl;
W is oxygen, -S- or NR(99)-;
R(99) is hydrogen or methyl;
or
R(3) and R(8)
are identical and are -SR(29), -OR(30), -NR(31)R(32) or -CR(33)R(34)R(35);
R(29), R(30), R(31) and R(33)
independently of one another are -CₐH₂ₐ-[furanyl, thienyl, pyrrolyl, imidazolyl, pyrazolyl, triazolyl, tetrazolyl, oxazolyl, isoxazolyl, thiazolyl, isothiazolyl, pyridyl, pyrazinyl, pyrimidinyl, pyridazinyl, indolyl, indazolyl, quinolyl, isoquinolyl, phthalazinyl, quinoxalinyl, cuinazolinyl or cinnolinyl],
which are unsubstituted or substituted by 1-2 substituents from the group consisting of F, Cl, CF₃, CH₃, methoxy, hydroxyl, amino, methylamino and dimethylamino;
a is zero or 1;
R(32), R(34) and R(35)
independently of one another are hydrogen or CH₃;
or
R(3) and R(8)
are identical and are R(72)-SO₂ or R(73)R(74)N-SO₂-;
R(72) is (C₁-C₄)-alkyl, CF₃, (C₃-C₄)-alkenyl or -CₛH₂ₛ-R(75);
s is zero or 1;
R(75) is (C₃-C₆)-cycloalkyl or phenyl,
which is not substituted or is substituted by 1-2 substituents from the group consisting of F, Cl, CF₃, methyl, methoxy and NR(76)R(77);
R(76) and R(77)
are hydrogen or CH₃,
R(73) is hydrogen, (C₁-C₄)-alkyl, CF₃, (C₃-C₄)-alkenyl or -C_{w}H_{2w}-R(78);
w is zero or 1;
R(78) is (C₃-C₆)-cycloalkyl or phenyl,
which is not substituted or is substituted by 1-2 substituents from the group consisting of F, Cl, CF₃, methyl, methoxy and NR(79)R(80);
R(79) and R(80)
are hydrogen or CH₃;
R(74) is hydrogen or CH₃;
or
R(73) and R(74)
together are 4 or 5 methylene groups,
of which one CH₂ group can be replaced by oxygen, S, NH, N-CH₃ or N-benzyl;
or
R(3) and R(8)
are identical and are R(39)X-;
X is oxygen, S, NR(40), (C=O)A- or NR(41)C=MN⁽*⁾R(42)-,
M is oxygen;
A is oxygen or NR(43);
R(43) is hydrogen, (C₁-C₄)-alkyl or (C₁-C₄)-perfluoroalkyl;
R(41) is hydrogen, (C₁-C₄)-alkyl;
R(42) is defined as R(39);
R(39) is (C₁-C₆)-alkyl, (C₃-C₄)-alkenyl, (CH₂)_{b}C_{d}F_{2d+1} or -CₓH₂ₓ-R(44);
b is zero or 1;
d is 1 - 7;
x is zero or 1,
R(44) is (C₃-C₆)-cycloalkyl or phenyl,
which is not substituted or is substituted by 1-2 substituents from the group consisting of F, Cl, CF₃, methyl, methoxy and NR(45)R(46);
(R45) and R(46)
are hydrogen or CH₃;
or
R(39) and R(40) or R(39) and R(41)
together are 4 or 5 methylene groups,
of which one CH₂ group can be replaced by oxygen, S, NH, N-CH₃ or N-benzyl;
where A and N⁽*⁾ are bonded to the phenyl nucleus of the benzoylguanidine parent structure;
or
R(3) and R(8)
are identical and are -SR(47), -OR(48), -NHR(49), -NR(50)R(51), -CHR(52)R(53), -CR(54)R(55)OH, -C≡C-R(56), -CR(58)=CHR(57) or-[CR(59)R(60)]ᵤ-CO-[CR(61)R(62)]ᵥ-R(63);
R(47), R(48), R(49), R(50) and R(52)
are identical or different and are -(CH₂)_{y}-(CHOH)_{z}-(CH₂)ₐₐ-(CHOH)ₜ-R(64) or -(CH₂)_{ab}-O-(CH₂-CH₂O)_{ac}-R(65);
y, z, aa, ab, ac
are identical or different and are zero, 1 or 2;
R(64) and R(65)
are hydrogen or methyl;
u is 1 or 2;
v is zero, 1 or 2;
t is 1, 2 or 3;
R(51), R(53), R(54) and R(55)
are identical or different and are hydrogen or methyl;
or
R(52) and R(53) or R(54) and R(55)
together with the carbon atom carrying them are a (C₃-C₆)-cycloalkyl,
R(63) is hydrogen, (C₁-C₄)-alkyl, (C₃-C₆)-cycloalkyl or -CₑH₂ₑ-R(81);
e is zero, 1 or 2;
R(56), R(57), R(81)
independently of one another are phenyl,
which is unsubstituted or is substituted by 1-2 substituents from the group consisting of F, Cl, CF₃, methyl, methoxy or NR(65)R(66) where R(65) and R(66) are identical and are hydrogen or CH₃;
or
R(56), R(57) and R(81)
independently of one another are furanyl, thienyl, pyrrolyl, imidazolyl, pyrazolyl, triazolyl, tetrazolyl, oxazolyl, isoxazolyl, thiazolyl, isothiazolyl, pyridyl, pyrazinyl, pyrimidinyl, pyridazinyl, indolyl, indazolyl, guinolyl, isoquinolyl, phthalazinyl, guinoxalinyl, quinazolinyl or cinnolinyl,
which are unsubstituted or substituted as phenyl;
R(58), R(59), R(60), R(61) and R(62)
are hydrogen, methyl;
or
R(3) and R(8)
are R(67)-NH-SO₂-;
R(67) is R(68)R(69)N-(C=Y');
Y' is oxygen, S or N-R(70);
R(68) and R(69)
are identical or different and are hydrogen, (C₁-C₄)-alkyl, (C₃-C₄)-alkenyl or -C_{f}H_{2f}-R(71);
f is zero or 1;
R(71) is (C₅-C₇)-cycloalkyl or phenyl,
which is unsubstituted or is substituted by 1-2 substituents from the group consisting of F, Cl, CF₃, methoxy and methyl;
or
R(68) and R(69)
together are 4 or 5 methylene groups,
of which one CH₂ group can be replaced by oxygen, S, NH, N-CH₃ or N-benzyl,
R(70) is defined as R(68);
R(4) and R(7)
are identical and are (C₁-C₈)-alkyl, -CₐₗH₂ₐₗR(84) or CF₃;
al is zero or 1;
R(84) is (C₃-C₆)-cycloalkyl or phenyl,
which is not substituted or is substituted by 1-2 substituents from the group consisting of F, Cl, CF₃, methyl, methoxy and NR(85)R(86);
R(85) and R(86)
are hydrogen or CH₃;
or
R(4) and R(7)
are identical and are quinolyl, isoquinolyl, pyrrolyl, pyridyl or imidazolyl
which are linked via C or N and which are unsubstituted or substituted by 1-2 substituents from the group consisting of F, Cl, CF₃, CH₃, methoxy, hydroxyl, amino, methylamino and dimethylamino;
or
R(4) and R(7)
are identical and are R(87)-SOₐₘ or R(88)R(89)N-SO₂-;
am is 2;
R(87) is (C₁-C₄)-alkyl or CF₃;
R(88) is hydrogen, (C₁-C₄)-alkyl, CF₃ or -CₐₙH₂ₐₙ-R(90),
an is zero or 1;
R(90) is phenyl,
which is not substituted or is substituted by 1-2 substituents from the group consisting of F, Cl, CF₃, methyl, methoxy and NR(90)R(91);
R(90) and R(91)
are hydrogen or CH₃;
R(89) is hydrogen or CH₃;
or
R(4) and R(7)
are identical and are -C≡CR(93);
R(93) is phenyl,
which is unsubstituted or substituted by 1-2 substituents from the group consisting of F, Cl, CF₃, methyl, methoxy and NR(94)R(95);
R(94) and R(95)
are hydrogen or CH₃;
R(5) and R(6)
are identical and are hydrogen;
or its pharmaceutically tolerable salts.

4. A process for preparing a compound I a as claimed in claim 1, wherein
a) in each case one equivalent of the compounds of the formula II aa and II bb is reacted with one equivalent of guanidine, 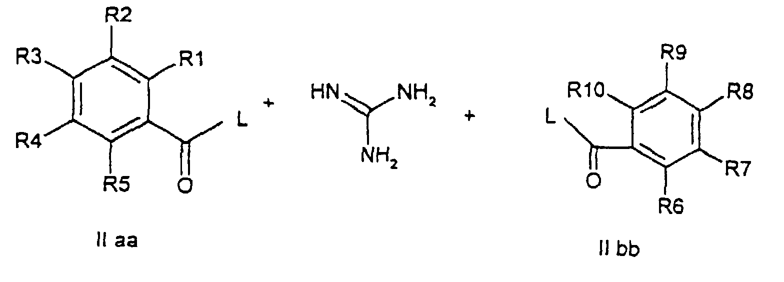 where R(1) to R(10) have the given meaning and L is a leaving group which can readily be substituted nucleophilically, or
b) reacting a compound of the formula II aa 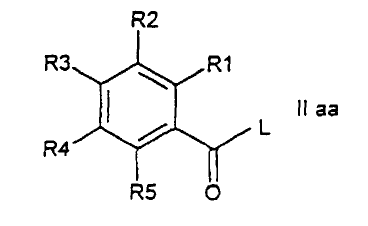 with a compound of the formula III aa 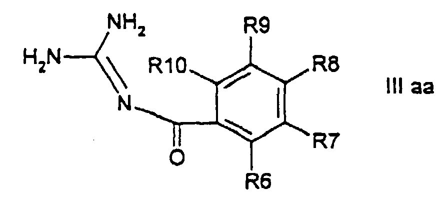 with the participation of a base, where R(1) to R(10) have the given meanings and L is a leaving group which can readily be substituted nucleophilically,
and converting, where appropriate, into a pharmaceutically tolerated salt.

5. The use of a compound I a as claimed in claim 1 for preparing a medicament for the treatment of arrhythmias.

6. The use of a compound I a as claimed in claim 1 for preparing a medicament for the treatment or prophylaxis of cardiac infarction.

7. The use of a compound I a as claimed in claim 1 for preparing a medicament for the treatment or prophylaxis of angina pectoris.

8. The use of a compound I a as claimed in claim 1 for preparing a medicament for the treatment or prophylaxis of ischemic conditions of the heart.

9. The use of a compound I a as claimed in claim 1 for preparing a medicament for the treatment or prophylaxis of ischemic conditions of the peripheral and central nervous system and of stroke.

10. The use of a compound I a as claimed in claim 1 for preparing a medicament for the treatment or prophylaxis of ischemic conditions of peripheral organs and limbs.

11. The use of a compound I a as claimed in claim 1 for preparing a medicament for the treatment of shock conditions.

12. The use of a compound I a as claimed in claim 1 for preparing a medicament for employment in surgical operations and organ transplantations.

13. The use of a compound I a as claimed in claim 1 for preparing a medicament for the preservation and storage of transplants for surgical procedures.

14. The use of a compound I a as claimed in claim 1 for preparing a medicament for the treatment of diseases in which cell proliferation represents a primary or secondary cause.

15. A medicine containing an effective quantity of a compound I a as claimed in claim 1.

## Revendications

1. Dibenzoylguanidines de la formule I a : 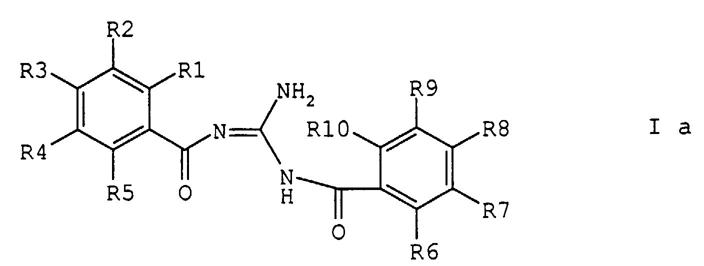 dans laquelle les significations des symboles sont :
R(1), R(5), R(6) et R(10)
indépendamment les uns des autres hydrogène, F, Cl, alkyle en (C₁-C₃), -OR(11), CᵣF₂ᵣ₊₁ ou -NR(11)R(12) ;
R(11) et R(12)
indépendamment l'un de l'autre hydrogène ou alkyle en (C₁-C₃) ;
r 1 à 4 ;
R(2), R(4), R(7) et R(9)
indépendamment les uns des autres hydrogène, F, Cl, Br, I, -C≡N, Xₒ-(CH₂)ₚ-(CF₂)_{q}-CF₃, R(13)-SOₘ, R(14)R(15)N-CO-, R(16)-CO- ou R(17)R(18)N-SO₂ ;
X oxygène, S ou NR(19) ;
R(19) hydrogène ou alkyle en (C₁-C₃) ;
m 0, 1 ou 2 ;
o 0 ou 1 ;
p 0, 1 ou 2 ;
q 0, 1, 2, 3, 4, 5 ou 6 ;
R(13), R(14), R(16) et R(17)
indépendamment les uns des autres alkyle en (C₁-C₈), alcényle en (C₃-C₆), -CₙH₂ₙ-R(20) ou CF₃ ;
n 0, 1, 2, 3 ou 4 ;
R(20) cycloalkyle en (C₃-C₇) ou phényle,
non substitué ou substitué par 1 à 3 substituants du groupe comprenant F, Cl, CF₃, méthyle, méthoxy et NR(21)R(22) ;
R(21) et R(22)
H ou alkyle en C₁-C₄ ;
ou bien
R(14), R(16) et R(17)
également H ;
R(15) et R(18)
indépendamment l'un de l'autre hydrogène ou alkyle en (C₁-C₄) ;
ou bien
R(14) et R(15) ainsi que R(17) et R(18)
forment ensemble 4 ou 5 groupes méthylène
desquels un groupe CH₂ peut être remplacé par un oxygène, S, NH, N-CH₃ ou N-benzyle ;
ou bien
R(2), R(4), R(7) et R(9)
indépendamment les uns des autres alkyle en (C₁-C₈) ou -CₐₗH₂ₐₗR(84) ;
al 0, 1 ou 2 ;
R(84) cycloalkyle en (C₃-C₈) ou phényle,
non substitué ou substitué par 1 à 3 substituants du groupe comprenant F, Cl, CF₃, méthyle, méthoxy et NR(85)R(86) ;
R(85) et R(86)
hydrogène ou CH₃ ;
ou bien
R(2), R(4), R(7) et R(9)
indépendamment les uns des autres, furanyle, thiényle, pyrrolyle, imidazolyle, pyrazolyle, triazolyle, tétrazolyle, oxazolyle, isoxazolyle, thiazolyle, isothiazolyle, pyridyle, pyrazinyle, pyrimidinyle, pyridazinyle, indolyle, indazolyle, quinolyle, isoquinolyle, phtalazinyle, quinoxalinyle, quinazolinyle ou cinnolinyle,
reliés par l'intermédiaire de C ou N et non substitués ou bien substitués par 1 à 3 substituants du groupe comprenant F, Cl, CF₃, CH₃, méthoxy, hydroxy, amino, méthylamino et diméthylamino ;
ou bien
R(2), R(4), R(7) et R(9)
indépendamment les uns des autres -C≡CR(93) ;
R(93) phényle,
non substitué ou bien substitué par 1 à 3 substituants du groupe comprenant F, Cl, CF₃, méthyle, méthoxy et NR(94)R(95) ;
R(94) et R(95)
hydrogène ou CH₃ ;
R(3) et R(8)
indépendamment l'un de l'autre définis comme R(2);
ou bien
R(3)et R(8)
indépendamment l'un de l'autre -Y-(p-C₆H₄)-(CO)ₕ-(CHOH)ᵢ-(CH₂)ⱼ-(CHOH)ₖ-R(23), -Y-(m-C₆H₄)-(CO)_{ad}-(CHOH)ₐₑ-(CH₂)_{af}-(CHOH)_{ag}-R(24) ou -Y-(o-C₆H₄)-(CO)ₐₕ-(CHOH)ₐₒ-(CH₂)ₐₚ-(CHOH)ₐₖ-(R25) ;
Y oxygène, -S- ou -NR(83)- ;
h, ad, ah
indépendamment les uns des autres 0 ou 1 ;
i, j, k, ae, af, ag, ao, ap et ak
indépendamment les uns des autres 0, 1, 2, 3 ou 4,
à la condition toutefois que, respectivement,
h, i et k ne valent pas simultanément 0 ;
ad, ae et ag ne valent pas simultanément 0 ;
ah, ao et ak ne valent pas simultanément 0 ;
R(23), R(24), R(25) et R(83)
indépendamment les uns des autres hydrogène ou alkyle en (C₁-C₃) ;
ou bien
R(3) et R(8)
indépendamment l'un de l'autre hydrogène, F, Cl, Br, I, CN, alkyle en (C₁-C₈), perfluoroalkyle en (C₁-C₈), alcényle en (C₃-C₈), -C_{g}H_{2g}R(26) ;
g 0, 1, 2, 3 ou 4 ;
R(26) cycloalkyle en (C₃-C₈), phényle, biphénylyle ou naphtyle,
les aromatiques étant non substitués ou substitués par 1 à 3 substituants du groupe F, Cl, CF₃, méthyle, méthoxy et NR(27)R(28) ;
R(27) et R(28)
hydrogène, alkyle en (C₁-C₄) ou perfluoroalkyle en (C₁-C₄) ;
ou bien
R(3) et R(8)
indépendamment l'un de l'autre SR(29), -OR(30), -NR(31)R(32) ou -CR(33)R(34)R(35) ;
R(29), R(30), R(31) et R(33)
indépendamment les uns des autres -CₐH₂ₐ-[furanyle, thiényle, pyrrolyle, imidazolyle, pyrazolyle, triazolyle, tétrazolyle, oxazolyle, isoxazolyle, thiazolyle, isothiazolyle, pyridyle, pyrazinyle, pyrimidinyle, pyridazinyle, indolyle, indazolyle, quinolyle, isoquinolyle, phtalazinyle, quinoxalinyle, quinazolinyle ou cinnolinyle],
non substitués ou bien substitués par 1 à 3 substituants du groupe comprenant F, Cl, CF₃, CH₃, méthoxy, hydroxy, amino, méthylamino, diméthylamino ou benzyle ;
a 0, 1 ou 2 ;
R(32), R(34) et R(35)
indépendamment les uns des autres définis comme R(29) ou hydrogène, alkyle en (C₁-C₄) ou perfluoroalkyle en (C₁-C₄) ;
ou bien
R(3) et R(8)
indépendamment l'un de l'autre -W-(p-C₆H₄)-R(96), -W-(m-C₆H₄)-R(97) ou -W-(o-C₆H₄)-R(98) ;
R(96), R(97) et R(98)
indépendamment les uns des autres furanyle, thiényle, pyrrolyle, imidazolyle, pyrazolyle, triazolyle, tétrazolyle, oxazolyle, isoxazolyle, thiazolyle, isothiazolyle, pyridyle, pyrazinyle, pyrimidinyle, pyridazinyle, indolyle, indazolyle, quinolyle, isoquinolyle, phtalazinyle, quinoxalinyle, quinazolinyle ou cinnolinyle,
reliés par l'intermédiaire de C ou N et non substitués ou bien substitués par 1 à 3 substituants du groupe comprenant F, Cl, CF₃, CH₃, méthoxy, hydroxy, amino, méthylamino, diméthylamino ou benzyle ;
W oxygène, S ou NR(99)- ;
R(99) hydrogène ou alkyle en (C₁-C₄) ;
ou bien
R(3) et R(8)
indépendamment l'un de l'autre R(72)-SOₘ ou R(73)R(74)N-SO₂- ;
m 1 ou 2 ;
R(72) alkyle en (C₁-C₈), perfluoroalkyle en (C₁-C₈), alcényle en (C₃-C₈) ou -CₛH₂ₛ-R(75) ;
s 0, 1, 2, 3 ou 4 ;
R(75) cycloalkyle en (C₃-C₈), phényle, biphénylyle ou naphtyle,
les aromatiques étant non substitués ou bien substitués par 1 à 3 substituants du groupe F, Cl, CF₃, méthyle, méthoxy et NR(76)R(77) ;
R(76) et R(77)
hydrogène, alkyle en (C₁-C₄) ou perfluoroalkyle en (C₁-C₄) ;
R(73) hydrogène, alkyle en (C₁-C₈), perfluoroalkyle en (C₁-C₈), alcényle en (C₃-C₈) ou -C_{w}H_{2w}-R(78) ;
w 0, 1, 2, 3 ou 4 ;
R(78) cycloalkyle en (C₃-C₈), phényle, biphénylyle ou naphtyle,
les aromatiques étant non substitués ou bien substitués par 1 à 3 substituants du groupe F, Cl, CF₃, méthyle, méthoxy et NR(79)R(80) ;
R(79) et R(80)
hydrogène, alkyle en (C₁-C₄) ou perfluoroalkyle en (C₁-C₄) ;
R(74) hydrogène, alkyle en (C₁-C₄) ou perfluoroalkyle en (C₁-C₄) ;
ou bien
R(73) et R(74)
forment ensemble 4 ou 5 groupes méthylène, desquels un groupe CH₂ peut être remplacé par un oxygène, S, NH, N-CH₃ ou N-benzyle ;
ou bien
R(3) et R(8)
indépendamment l'un de l'autre R(39)X- ;
X oxygène, S, NR(40), (D=O)A-, NR(41)C=MN⁽*⁾R(42)- ;
M oxygène ou S ;
A oxygène ou NR(43) ;
D C ou SO ;
R(39) alkyle en (C₁-C₈), alcényle en (C₃-C₈), (CH₂)_{b}C_{d}F_{2d+1} ou -CₓH₂ₓ-R(44) ;
b 0 ou 1 ;
d 1, 2, 3, 4, 5, 6 ou 7 ;
x 0, 1, 2, 3 ou 4 ;
R(44) cycloalkyle en (C₃-C₈), phényle, biphénylyle ou naphtyle,
les aromatiques étant non substitués ou bien substitués par 1 à 3 substituants du groupe F, Cl, CF₃, méthyle, méthoxy et NR(45)R(46) ;
R(45) et R(46)
hydrogène, alkyle en (C₁-C₄) ou perfluoroalkyle en (C₁-C₄) ;
R(40), R(41) et R(43)
indépendamment les uns des autres hydrogène, alkyle en (C₁-C₄) ou perfluoroalkyle en (C₁-C₄) ;
R(42) défini comme R(39) ;
ou
R(39) et R(40) respectivement R(39) et R(41)
forment ensemble 4 ou 5 groupes méthylène,
desquels un groupe CH₂ peut être remplacé par un oxygène, S, NH, N-CH₃ ou N-benzyle ;
A et N⁽*⁾ étant liés au noyau phényle de la substance de base benzoylguanidine ;
ou bien
R(3) et R(8)
indépendamment l'un de l'autre -SR(47), -OR(48), -NHR(49), -NR(50)R(51), -CHR(52)R(53), -CR(54)R(55)OH, -C≡C-R(56), -CR(58)=CHR(57) ou -[CR(59)R(60)]ᵤ-CO-[CR(61)R(62)]ᵥ-R(63) ;
R(47), R(48), R(49), R(50) et R(52) identiques ou différents -(CH₂)_{y}-(CHOH)_{z}-(CH₂)ₐₐ-(CHOH)ₜ-R(64) ou -(CH₂)_{ab}-O-(CH₂-CH₂O)_{ac}-R(65) ;
R(64) et R(65)
hydrogène ou méthyle ;
u 1, 2, 3 ou 4 ;
v 0, 1, 2, 3 ou 4 ;
y, z et aa
identiques ou différents, 0, 1, 2, 3 ou 4 ;
t 1, 2, 3 ou 4 ;
R(51), R(53), R(54) et R(55)
identiques ou différents, hydrogène ou alkyle en (C₁-C₆) ;
ou bien
R(52) et R(53) respectivement R(54) et R(55)
forment ensemble avec l'atome de carbone qui les porte un cycloalkyle en (C₃-C₈) ;
R(63) hydrogène, alkyle en (C₁-C₆), cycloalkyle en (C₃-C₈) ou -CₑH₂ₑ-R(81) ;
e 0, 1, 2, 3 ou 4 ;
R(56), R(57) et R(81)
indépendamment phényle,
non substitué ou bien substitué par 1 à 3 substituants du groupe F, Cl, CF₃, méthyle, méthoxy ou NR(82)R(66) avec R(82) et R(66) = H ou alkyle en (C₁-C₄);
ou bien
R(56), R(57) et R(81)
indépendamment les uns des autres furanyle, thiényle, pyrrolyle, imidazolyle, pyrazolyle, triazolyle, tétrazolyle, oxazolyle, isoxazolyle, thiazolyle, isothiazolyle, pyridyle, pyrazinyle, pyrimidinyle, pyridazinyle, indolyle, indazolyle, quinolyle, isoquinolyle, phtalazinyle, quinoxalinyle, quinazolinyle ou cinnolinyle,
non substitués ou bien substitués comme le phényle ;
R(58), R(59), R(60), R(61) et R(62)
hydrogène ou méthyle ;
ou bien
R(3) et R(8)
indépendamment l'un de l'autre R(67)-NH-SO₂- ;
R(67) R(68)R(69)N-(C=Y')- ;
Y' oxygène, S ou N-R(70) ;
R(68) et R(69)
identiques ou différents, hydrogène, alkyle en (C₁-C₈), alcényle en (C₃-C₆) ou -C_{f}H_{2f}-R(71) ;
f 0, 1, 2, 3 ou 4 ;
R(71) cycloalkyle en (C₅-C₇) ou phényle,
non substitué ou substitué par 1 à 3 substituants du groupe F, Cl, CF₃, méthoxy et alkyle en (C₁-C₄) ;
ou bien
R(68) et R(69)
forment ensemble 4 ou 5 groupes méthylène
desquels un groupe CH₂ peut être remplacé par un oxygène, S, NH, N-CH₃ ou N-benzyle ;
avec
R(70) comme R(68) ou amidine ;
ainsi que leurs sels pharmaceutiquement acceptables,
étant toutefois exclus les composés dans lesquels les résidus R(1) à R(10) sont combinés comme suit :
| R(1) | R(2) | R(3) | R(4) | R(5) | R(6) | R(7) | R(8) | R(9) | R(10) |
|---|---|---|---|---|---|---|---|---|---|
| H | Cl | Cl | H | H | H | H | Cl | Cl | H |
| H | H | NH₂ | H | H | H | H | NH₂ | H | H |
| H | H | H | H | H | H | H | H | H | H |
| Cl | H | H | H | H | H | H | H | H | Cl |
| H | H | Cl | H | H | H | H | H | H | H |
| H | H | CH₃ | H | H | H | H | CH₃ | H | H |
| H | H | NH₂ | H | H | H | H | H | H | H |
| H | H | Cl | H | H | H | H | H | H | H |
| H | H | CH₃ | H | H | H | H | H | H | H |

2. Composé de la formule I a selon la revendication 1, dans laquelle :
R(5) et R(6)
sont des hydrogènes ;
les autres résidus sont tels que définis dans la revendication 1 ;
et
R(1) et R(10) sont identiques ;
R(2) et R(9) sont identiques ;
R(3) et R(8) sont identiques ;
R(4) et R(7) sont identiques.

3. Composés de la formule I a selon la revendication 1, dans lesquels les significations des symboles sont :
R(1) et R(10)
identiques, hydrogène, F, Cl, CH₃, OH, NH₂ ou CF₃ ;
R(2) et R(9)
identiques, hydrogène, F, Cl, Br, -C≡N, -C_{q}H_{2q}CF₃, R(13)-SO₂, R(14)R(15)N-CO-, R(16)-CO- ou R(17)R(18)N-SO₂- ;
q 0, 1, 2, 3, 4 ou 5 ;
R(13), R(14), R(16) et R(17)
indépendamment les uns des autres alkyle en (C₁-C₈), alcényle en (C₃-C₄), -CₙH₂ₙ-R(20) ou CF₃ ;
n 0 ou 1 ;
R(20) cycloalkyle en (C₃-C₆) ou phényle,
non substitué ou bien substitué par 1 à 2 substituants du groupe F, Cl, CF₃, méthyle, méthoxy et NR(21)R(22) ;
R(21) et R(22)
hydrogène ou méthyle ;
ou bien R(14), R(16) et R(17)
hydrogène ;
R(15) et R(18)
indépendamment l'un de l'autre hydrogène ou méthyle ;
R(3) et R(8)
identiques, -Y-(p-C₆H₄)-(CO)ₕ-(CHOH)ᵢ-(CH₂)ⱼ-(CHOH)ₖ-R(23), -Y-(m-C₆H₄)-(CO)_{ad}-(CHOH)ₐₑ-(CH₂)_{af}-(CHOH)_{ag}-R(24) ou -Y-(o-C₆H₄)-(CO)ₐₕ-(CHOH)ₐₒ-(CH₂)ₐₚ-(CHOH)ₐₖ-(R25) ;
Y oxygène, S ou -NR(83) ;
R(23), R(24), R(25) et R(83)
indépendamment les uns des autres hydrogène ou méthyle ;
h, ad et ah
indépendamment les uns des autres 0 ou 1 ;
i, k, ae, ag, ao et ak
indépendamment les uns des autres 0, 1, 2 ou 3 ;
j, af et ap
indépendamment les uns des autres 0 ou 1 ;
à la condition toutefois que, respectivement,
h, i et k ne valent pas simultanément 0 ;
ad, ae et ag ne valent pas simultanément 0 ;
ah, ao et ak ne valent pas simultanément 0 ;
ou bien
R(3) et R(8)
identiques, hydrogène, F, Cl, Br, CN, alkyle en (C₁-C₈), CF₃, alcényle en (C₃-C₈) ou -C_{g}H_{2g}R(26) ;
g 0, 1 ou 2 ;
R(26) cycloalkyle en (C₃-C₆) ou phényle,
non substitué ou bien substitué par 1 à 2 substituants du groupe F, Cl, CF₃, méthyle, méthoxy et NR(27)R(28) ;
R(27) et R(28)
H ou CH₃ ;
ou bien
R(3) et R(8)
identiques, furanyle, thiényle, pyrrolyle, imidazolyle, pyrazolyle, triazolyle, tétrazolyle, oxazolyle, isoxazolyle, thiazolyle, isothiazolyle, pyridyle, pyrazinyle, pyrimidinyle, pyridazinyle, indolyle, indazolyle, quinolyle, isoquinolyle, phtalazinyle, quinoxalinyle, quinazolinyle ou cinnolinyle,
reliés par l'intermédiaire de C ou N et non substitués ou bien substitués par 1 ou 2 substituants du groupe comprenant F, Cl, CF₃, CH₃, méthoxy, hydroxy, amino, méthylamino et diméthylamino ;
ou bien
R(3) et R(8)
identiques, -W-(p-C₆H₄)-R(96), -W-(m-C₆H₄)-R(97) ou -W-(o-C₆H₄)-R(98) ;
R(96), R(97) et R(98)
indépendamment les uns des autres pyrrolyle, imidazolyle, pyrazolyle ou pyridyle,
respectivement non substitué ou bien substitué par 1 à 2 résidus de la série F, Cl, CF₃, CH₃, méthoxy, diméthylamino et benzyle ;
W oxygène, -S- ou NR(99)- ;
R(99) hydrogène ou méthyle ;
ou bien
R(3) et R(8)
identiques, -SR(29), -OR(30), -NR(31)R(32) ou -CR(33)R(34)R(35) ;
R(29), R(30), R(31) et R(33)
indépendamment les uns des autres -CₐH₂ₐ-[furanyle, thiényle, pyrrolyle, imidazolyle, pyrazolyle, triazolyle, tétrazolyle, oxazolyle, isoxazolyle, thiazolyle, isothiazolyle, pyridyle, pyrazinyle, pyrimidinyle, pyridazinyle, indolyle, indazolyle, quinolyle, isoquinolyle, phtalazinyle, quinoxalinyle, quinazolinyle ou cinnolinyle],
non substitués ou bien substitués par 1 à 2 substituants du groupe comprenant F, Cl, CF₃, CH₃, méthoxy, hydroxy, amino, méthylamino et diméthylamino ;
a 0, 1 ou 2 ;
R(32), R(34) et R(35)
indépendamment les uns des autres hydrogène ou CH₃ ;
ou bien
R(3) et R(8)
identiques, R(72)-SO₂ ou R(73)R(74)N-SO₂- ;
R(72) alkyle en (C₁-C₄), CF₃, alcényle en (C₃-C₄) ou -CₛH₂ₛ-R(75) ;
s 0 ou 1 ;
R(75) cycloalkyle en (C₃-C₆) ou phényle,
non substitué ou bien substitué par 1 à 2 substituants du groupe F, Cl, CF₃, méthyle, méthoxy et NR(76)R(77) ;
R(76) et R(77)
hydrogène ou CH₃ ;
R(73) hydrogène, alkyle en (C₁-C₄), CF₃, alcényle en (C₃-C₄) ou -C_{w}H_{2w}-R(78) ;
w 0 ou 1 ;
R(78) cycloalkyle en (C₃-C₆) ou phényle,
non substitué ou bien substitué par 1 à 2 substituants du groupe F, Cl, CF₃, méthyle, méthoxy et NR(79)R(80) ;
R(79) et R(80)
hydrogène ou CH₃ ;
R(74) hydrogène ou CH₃ ;
ou bien
R(73) et R(74)
forment ensemble 4 ou 5 groupes méthylène,
desquels un groupe CH₂ peut être remplacé par un oxygène, S, NH, N-CH₃ ou N-benzyle ;
ou bien
R(3) et R(8)
identiques = R(39)X- ;
X oxygène, S, NR(40), (C=O)A- ou NR(41)C=MN⁽*⁾R(42)- ;
M oxygène ;
A oxygène ou NR(43) ;
R(43) hydrogène, alkyle en (C₁-C₄) ou perfluoroalkyle en (C₁-C₄) ;
R(41) hydrogène, alkyle en (C₁-C₄) ;
R(42) défini comme R(39) ;
R(39) alkyle en (C₁-C₆), alcényle en (C₃-C₄), (CH₂)_{b}C_{d}F_{2d+1} ou -CₓH₂ₓ-R(44) ;
b 0 ou 1 ;
d 1 à 7 ;
x 0 ou 1 ;
R(44) cycloalkyle en (C₃-C₆) ou phényle,
non substitué ou bien substitué par 1 à 2 substituants du groupe F, Cl, CF₃, méthyle, méthoxy et NR(45)R(46) ;
R(45) et R(46)
hydrogène ou CH₃ ;
ou bien
R(39) et R(40) respectivement R(39) et R(41) forment ensemble 4 ou 5 groupes méthylène,
desquels un groupe CH₂ peut être remplacé par un oxygène, S, NH, N-CH₃ ou N-benzyle ;
A et N⁽*⁾ étant liés au noyau phényle de la substance de base benzoylguanidine ;
ou bien
R(3) et R(8)
identiques = -SR(47), -OR(48), -NHR(49), -NR(50)R(51), -CHR(52)R(53), -CR(54)R(55)OH, -C≡C-R(56), -CR(58)=CHR(57) ou -[CR(59)R(60)]ᵤ-CO-[CR(61)R(62)]ᵥ-R(63) ;
R(47), R(48), R(49), R(50) et R(52)
identiques ou différents -(CH₂)_{y}-(CHOH)_{z}-(CH₂)ₐₐ-(CHOH)ₜ-R(64) ou -(CH₂)_{ab}-O-(CH₂-CH₂O)_{ac}-R(65) ;
y, z, aa, ab, ac
identiques ou différents, 0, 1 ou 2 ;
R(64) et R(65)
hydrogène ou méthyle ;
u 1 ou 2 ;
v 0, 1 ou 2 ;
t 1, 2 ou 3 ;
R(51), R(53), R(54) et R(55)
identiques ou différents, hydrogène ou méthyle ;
ou bien
R(52) et R(53) respectivement R(54) et R(55) forment ensemble avec l'atome de carbone qui les porte un cycloalkyle en (C₃-C₆) ;
R(63) hydrogène, alkyle en (C₁-C₄), cycloalkyle en (C₃-C₆) ou -CₑH₂ₑ-R(81) ;
e 0, 1 ou 2 ;
R(56), R(57), R(81)
indépendamment les uns des autres phényle,
non substitué ou bien substitué par 1 à 2 substituants du groupe F, Cl, CF₃, méthyle, méthoxy ou NR(65)R(66) avec R(65) et R(66) identiques, hydrogène ou CH₃ ;
ou bien
R(56), R(57) et R(81)
indépendamment les uns des autres furanyle, thiényle, pyrrolyle, imidazolyle, pyrazolyle, triazolyle, tétrazolyle, oxazolyle, isoxazolyle, thiazolyle, isothiazolyle, pyridyle, pyrazinyle, pyrimidinyle, pyridazinyle, indolyle, indazolyle, quinolyle, isoquinolyle, phtalazinyle, quinoxalinyle, quinazolinyle ou cinnolinyle
non substitués ou bien substitués comme le phényle ;
R(58), R(59), R(60), R(61) et R(62)
hydrogène, méthyle ;
ou bien
R(3) et R(8)
R(67)-NH-SO₂- ;
R(67) R(68)R(69)N-(C=Y')- ;
Y' oxygène, S ou N-R(70) ;
R(68) et R(69)
identiques ou différents, hydrogène, alkyle en (C₁-C₄), alcényle en (C₃-C₄) ou -C_{f}H_{2f}-R(71) ;
f 0 ou 1 ;
R(71) cycloalkyle en (C₅-C₇) ou phényle
non substitué ou substitué par 1 à 2 substituants du groupe F, Cl, CF₃, méthoxy et méthyle ;
ou bien
R(68) et R(69)
forment ensemble 4 ou 5 groupes méthylène
desquels un groupe CH₂ peut être remplacé par un oxygène, S, NH, N-CH₃ ou N-benzyle ;
R(70) défini comme R(68) ;
R(4) et R(7)
identiques, alkyle en (C₁-C₈), -CₐₗH₂ₐₗR(84) ou CF₃ ; al 0 ou 1 ;
R(84) cycloalkyle en (C₃-C₆) ou phényle,
non substitué ou bien substitué par 1 à 2 substituants du groupe comprenant F, Cl, CF₃, méthyle, méthoxy et NR(85)R(86) ;
R(85) et R(86)
hydrogène ou CH₃ ;
ou bien
R(4) et R(7)
identiques, quinolyle, isoquinolyle, pyrrolyle, pyridyle ou imidazolyle,
reliés par l'intermédiaire de C ou N et non substitués ou bien substitués par 1 à 2 substituants du groupe comprenant F, Cl, CF₃, CH₃, méthoxy, hydroxy, amino, méthylamino et diméthylamino ;
ou bien
R(4) et R(7)
identiques, R(87)-SOₐₘ ou R(88)R(89)N-SO₂- ;
am 2 ;
R(87) alkyle en (C₁-C₄) ou CF₃ ;
R(88) hydrogène, alkyle en (C₁-C₄), CF₃ ou -CₐₙH₂ₐₙ-R(90) ;
an 0 ou 1 ;
R(90) phényle,
non substitué ou bien substitué par 1 à 2 substituants du groupe comprenant F, Cl, CF₃, méthyle, méthoxy et NR(90)R(91) ;
R(90) et R(91)
hydrogène ou CH₃ ;
R (89) hydrogène ou CH₃
ou bien
R(4) et R(7)
identiques, -C≡CR(93) ;
R(93) phényle,
non substitué ou bien substitué par 1 à 2 substituants du groupe comprenant F, Cl, CF₃, méthyle, méthoxy et NR(94)R(95) ;
R(94) et R(95)
hydrogène ou CH₃ ;
R(5) et R(6)
identiques, hydrogène ;
ainsi que leurs sels pharmaceutiquement acceptables.

4. Procédé de préparation d'un composé I a selon la revendication 1, caractérisé en ce que
a) on fait réagir un équivalent de chacun des composés des formules II aa et II bb avec un équivalent de guanidine, 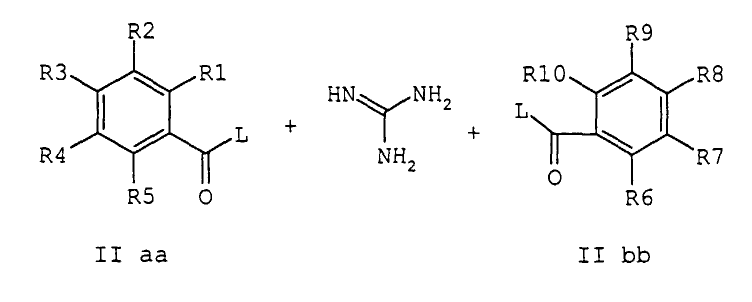 R(1) à R(10) ayant les significations indiquées et L représentant un groupe partant facilement substituable de manière nucléophile, ou bien
b) on fait réagir un composé de la formule II aa 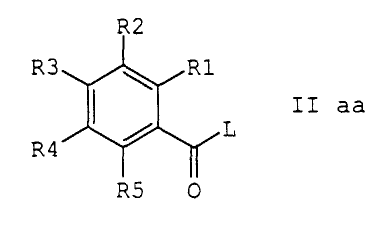 avec un composé de la formule III aa 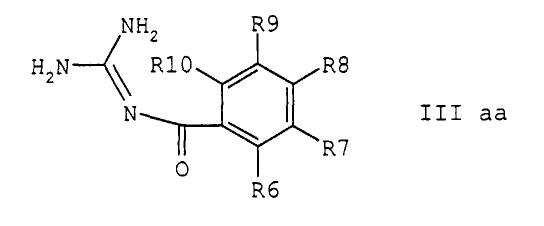 avec la participation d'une base, R(1) à R(10) ayant les significations indiquées et L représentant un groupe partant facilement substituable de manière nucléophile,
et le cas échéant on le convertit en un sel pharmaceutiquement compatible.

5. Utilisation d'un composé I a selon la revendication 1 pour préparer un médicament destiné au traitement des arythmies.

6. Utilisation d'un composé I a selon la revendication 1 pour préparer un médicament destiné au traitement ou à la prévention de l'infarctus du myocarde.

7. Utilisation d'un composé I a selon la revendication 1 pour préparer un médicament destiné au traitement ou à la prévention de l'angor.

8. Utilisation d'un composé I a selon la revendication 1 pour préparer un médicament destiné au traitement ou à la prévention des états ischémiques du coeur.

9. Utilisation d'un composé I a selon la revendication 1 pour préparer un médicament destiné au traitement ou à la prévention des états ischémiques du système nerveux périphérique et central et de l'apoplexie cérébrale.

10. Utilisation d'un composé I a selon la revendication 1 pour préparer un médicament destiné au traitement ou à la prévention des états ischémiques des organes périphériques et des membres.

11. Utilisation d'un composé I a selon la revendication 1 pour préparer un médicament destiné au traitement des états de choc.

12. Utilisation d'un composé I a selon la revendication 1 pour préparer un médicament destiné à être utilisé dans des interventions chirurgicales et des transplantations d'organes.

13. Utilisation d'un composé I a selon la revendication 1 pour préparer un médicament destiné à la conservation et au stockage de transplants pour des opérations chirurgicales.

14. Utilisation d'un composé I a selon la revendication 1 pour préparer un médicament destiné au traitement de maladies dans lesquelles la prolifération cellulaire représente une cause primaire ou secondaire.

15. Médicament contenant une quantité efficace d'un composé I a selon la revendication 1.
